(19) Europäisches Patentamt
European Patent Office
Office européen des brevets

(11) Veröffentlichungsnummer: **0 152 808 B1**

# (12) EUROPÄISCHE PATENTSCHRIFT

(45) Veröffentlichungstag der Patentschrift: **12.09.90**

(21) Anmeldenummer: **85100809.4**

(22) Anmeldetag: **26.01.85**

(51) Int. Cl.$^5$: **C 07 D 239/26,** C 07 D 239/30, C 07 D 239/34, C 09 K 19/00

## (54) Stickstoffhaltige Heterocyclen.

(30) Priorität: **07.02.84 DE 3404116**

(43) Veröffentlichungstag der Anmeldung: **28.08.85 Patentblatt 85/35**

(45) Bekanntmachung des Hinweises auf die Patenterteilung: **12.09.90 Patentblatt 90/37**

(84) Benannte Vertragsstaaten: **CH DE FR GB LI**

(56) Entgegenhaltungen:
**EP-A-0 014 885**
**EP-A-0 025 119**
**EP-A-0 056 501**
**EP-A-0 097 033**
**EP-A-0 104 011**
**DE-A-3 140 868**
**GB-A-2 092 169**

**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **Eidenschink, Rudolf, Dr.**
**Kornblumenstrasse 1**
**D-6115 Münster (DE)**
Erfinder: **Krause, Joachim, Dr.**
**Samuel-Morse-Strasse 14**
**D-6110 Dieburg (DE)**
Erfinder: **Hittich, Reinhard, Dr.**
**Am Kirchberg 11**
**D-6101 Modautal 1 (DE)**
Erfinder: **Poetsch, Eike, Dr.**
**Am Buchwald 4**
**D-6109 Mühltal 6 (DE)**
Erfinder: **Scheuble, Bernhard, Dr.**
**Am Grenzweg 18**
**D-6146 Alsbach (DE)**
Erfinder: **Weber, Georg**
**Wilhelm-Leuschner-Strasse 38**
**D-6106 Erzhausen (DE)**
Erfinder: **Pohl, Ludwig, Dr.**
**Niebergallweg 5**
**D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 152 808 B1

**Beschreibung**

Die Erfindung betrifft stickstoffhaltige Heterocyclen der Formel I

$$R^1—A^1—Z^1—A^2—Z^2—A^3—R^2$$

worin $R^1$ und $R^2$ jeweils unabhängig voneinander eine Alkylgruppe mit 1—15 C-Atomen, worin auch eine oder zwei nicht benachbarte $CH_2$-Gruppen durch O-Atome und/oder —CO-Gruppen und/oder —O—CO-Gruppen und/oder —CO—O-Gruppen ersetzt sein können, einer der Reste $R^1$ und $R^2$ auch H, F, Cl, Br oder CN,

$A^1$ eine 1,4-Cyclohexylengruppe, worin auch eine oder zwei nicht benachbarte $CH_2$-Gruppen durch O-Atome ersetzt sein können, eine 1,4-Bicyclo-(2,2,2)-octylengruppe oder eine 1,3-Dithian-2,5-diyl-Gruppe,

$Z^1$ und $Z^2$ jeweils unabhängig voneinander —$CH_2CH_2$—, —$OCH_2$—, —$CH_2O$— oder eine Einfachbindung,

$A^2$ und $A^3$ jeweils unabhängig voneinander eine 1,4-Phenylengruppe, Pyrimidin-2,5-diyl-gruppe, 1,4-Cyclohexylen-gruppe, worin auch eine oder zwei nicht benachbarte $CH_2$-Gruppen durch O-Atome ersetzt sein können, 1,3-Dithian-2,5-diylgruppe oder eine 1,4-Bicyclo(2,2,2)-octylengruppe bedeuten,
mit den Maßgaben, daß

(a) mindestens eine der Gruppen $A^2$ und $A^3$ eine Pyrimidin-2,5-diylgruppe ist,

(b) mindestens eine der Gruppen $Z^1$ und $Z^2$ keine Einfachbindung bedeutet, wenn $A^2$ Pyrimidin-2,5-diyl und $R^2$ Alkyl oder CN ist,

(c) 2-(4-Cyanphenyl)-5-[2-(trans-4-ethylcyclohexyl)-ethyl]-pyrimidin,
2-(4-Cyanphenyl)-5-[2-(trans-4-propylcyclohexyl)-ethyl]-pyrimidin,
2-(4-Cyanphenyl)-5-[2-(trans-4-butylcyclohexyl)-ethyl]-pyrimidin,
2-(4-Cyanphenyl)-5-[2-(trans-4-pentylcyclohexyl)-ethyl]-pyrimidin und
2-(4-Cyanphenyl)-5-[2-(trans-4-heptylcyclohexyl)-ethyl]-pyrimidin,
ausgeschlossen ist, und für die Ver tragstaceten CH DE GR LI.

(d) 5-Alkyl-2-(4'-(trans-4''-alkylcyclohexyl)phenyl)pyrimidine ausgeschlossen sind.

Der Einfachheit halber bedeuten im folgenden Cy eine 1,4-Cyclohexylengruppe, Dio eine 1,3-Dioxan-2,5-diylgruppe, Dit eine 1,3-Dithian-2,5-diylgruppe, Bi eine 1,4-Bicyclo(2,2,2)-octylengruppe, Phe eine 1,4-Phenylengruppe und Pyr eine Pyrimidin-2,5-diylgruppe.

Ähnliche Verbindungen sind z.B. aus der DE—PS 22 57 588 bekannt. Die dort angegebenen Verbindungen enthalten jedoch in Gegensatz zu den vorliegenden keine gesättigten Ringe (Cy, Dio, Dit, Bi).

Weiterhin sind aus der EP—A—0 025 119 5-Alkyl-2-(4-benzoyloxy- bzw. -cyclohexylcarbonyloxy-phenyl)-pyrimidine bekannt. Diese weisen im Gegensatz zu den erfindungsgemäßen Verbindungen eine Estergruppe (—COO—) zwischen zwei cyclischen Gruppen auf.

Aus der EP—A—0 014 885 und der DE—OS—3 140 868 sind 4-(5-Phenyl- bzw. -Cyclohexyl)-pyrimidin-2-yl)-benzol-Derivate bekannt. Die erfindungsgemäßen Verbindungen weisen dagegen entsprechend Maßgabe b) mindestens eine von einer Einfachbindung verschiedene Gruppe $Z^1$ und $Z^2$ auf, falls $R^2$ CN oder Alkyl bedeutet.

In der EP—A—0 056 501 und der GB—A—2 092 169 werden flüssigkristalline Mischungen beansprucht, welche Verbindungen enthalten, deren allgemeine Formel auch die erfindungsgemäßen Verbindungen umfaßt; dort werden jedoch lediglich 2-(p-Alkylphenyl)-5-cyano- bzw. -5-alkylpyrimidine beschrieben.

Der Anmelder hat sich unter Bezugnahme auf die ältere, nicht vorveröffentlichte Anmeldung EP—A—0 104 011 in den dort benannten Vertragsstaaten Schweiz, Bundesrepublik Deutschland, Großbritannien und Lichtenstein entsprechend Maßgabe d) eingeschränkt und gesondert Patentansprüche für den Vertragsstaat Frankreich vorgelegt.

Unter Bezugnahme auf die ältere, nicht vorveröffenlichte Anmeldung EP—A—0 149 208 hat sich der Anmelder in allen benannten Vertragsstaaten entsprechend der Maßgabe c) eingeschränkt.

Die Verbindungen der Formel I können wie ähnliche Verbindungen als Komponenten flüssigkristalliner Phasen verwendet werden, insbesondere für Displays, die auf dem Prinzip der verdrillten Zelle (TN-Displays), dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüsigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen für TN-Displays mit hohen Multiplexraten herstellbar.

Überraschenderweise zeigte sich beim Zusatz von Verbindungen der Formel I zu flüssigkristallinen Phasen, daß selbst größere Zusätze (z.B. von 10—30%) die Schwellenspannung nur unwesentlich erhöhen. Völlig unerwartet tritt gleichzeitig eine erhebliche Verbesserung der Kennliniensteilheit der Mischung auf, so daß Verbindungen des Typs I als besonders vorteilhaft geeignete Substanzen zur Herstellung von flüssig-kristallinen Mischungen mit steiler Kennlinie anzusehen sind.

Sie ermöglichen damit die Entwicklung hoch multiplexbarer Mischungen sehr kleiner optischer Anisotropie, mit denen eine Drehzelle insbesondere im ersten Transmissionsminimum nach Gooch-Tarry

betrieben werden kann. Damit ergbit sich eine sehr kleine Beobachtungswinkel-Abhängigkeit des Kontrastes.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eigen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppen und/oder C—C-Bindungen enthält, mit einem reduzierenden Mittel behandelt, oder daß man zur Herstellung von Estern der Formel I (worin $R^1$ und/oder $R^2$ eine Alkylgruppe bedeuten, worin eine oder zwei $CH_2$-Gruppen durch —O—CO-Gruppen und/oder —CO—O-Gruppen ersetzt sind) eine entsprechende Carbonsäure oder eines ihrer reaktionsfähigen Derivate mit einem entsprechenden Alkohol oder einem seiner reaktionsfähigen Derivate umsetzt, oder daß man zur Herstellung von 1,3-Dioxanderivaten bzw. 1,3-Dithianderivaten der Formel I (worin $A^1$ 1,3-Dioxan-2,5-diyl bzw. 1,3-Dithian-2,5-diyl bedeutet) einen entsprechenden Aldehyd mit einem entsprechenden Diol bzw. Dithiol umsetzt, oder daß man zur Herstellung von Ethern der Formel I (worin $R^1$ und/oder $R^2$ eine Alkylgruppe bedeuten, worin eine oder zwei $CH_2$-Gruppen durch O-Atome ersetzt sind und/oder Z ein $—OCH_2—$ oder $—CH_2—O$-Gruppe ist) eine entsprechende Hydroxyverbindung verethert), und/oder daß man gegebenenfalls eine Base der Formel I durch Behandeln mit einer Säure in eines ihrer Säureadditionssalze umwandelt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Weiterhin Gegenstand der Erfindung sind flüssigkristalline Phasen mit mindestens zwei flüssigkristallinen Komponenten dadurch gekennzeichnet, daß mindestens eine Komponente eine Verbindung der Formel I ist,

$$R^1—A^1—Z^1—A^2—Z^2—A^3—R^2 \qquad I$$

worin $R^1$, $R^2$, $A^1$, $A^2$, $A^3$, $Z^1$ und $Z^2$ die in Anspruch 1 angegebene Bedeutung besitzen mit den Maßgaben, daß

(a) mindestens eine der Gruppen $A^2$ und $A^3$ eine Pyrimidin-2,5-diylgruppe ist, und

(b) mindestens eine der Gruppen $Z^1$ und $Z^2$ keine Einfachbindung bedeutet, wenn $A^2$ Pyrimidin-2,5-diyl und $R^2$ Alkyl oder CN ist,

(c) 2-(4-Cyanphenyl)-5-[2-(trans-4-ethylcyclohexyl)-ethyl]-pyrimidin,
2-(4-Cyanphenyl)-5-[2-(trans-4-propylcyclohexyl)-ethyl]-pyrimidin,
2-(4-Cyanphenyl)-5-[2-(trans-4-butylcyclohexyl)-ethyl]-pyrimidin,
2-(4-Cyanphenyl)-5-[2-(trans-4-pentylcyclohexyl)-ethyl]-pyrimidin und
2-(4-Cyanphenyl)-5-[2-(trans-4-heptylcyclohexyl)-ethyl]-pyrimidin, ausgeschlossen ist, und sie mindestens einen Bestandteil der Formel II enthält

$$R'—L—G—E—R'' \qquad II$$

worin

L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinalozin gebildeten Gruppe,

| | | |
|---|---|---|
| G | —CH=CH— | —N(O)=N— |
| | —CH=CY— | —CH=N(O)— |
| | —CH≡C— | $—CH_2—CH_2—$ |
| | —CO—O— | $—CH_2—O—$ |
| | —CO—S— | $—CH_2—S—$ |
| | —CH=N— | —COO—Ph—COO— |

Y Halogen oder —CN, und

R' und R'' Alkoxy, Alkanoyloxy oder Alkoxycarbonyloxy mit bis zu 8 Kohlenstoffatomen, oder einer dieser Reste auch CN, NC, $NO_2$, $CF_3$, F, Cl oder Br bedeuten, oder sie mindestens einen anderen Bestandteil enthält, ausgewählt aus den Klassen der Benzylidenaniline, Terphenyl, Cyclohexylbiphenyl, Cyclohexylcyclohexane, Cyclohexylnaphthaline, 1,4-Bis-cyclohexylbenzole, 4,4'-Bis-cyclohexylbiphenyle, gegebenenfalls halogenierten Stilbene, Benzylphenylether, Tolane und substituierten Zimtsäuren.

Vor- und nachstehend haben $R^1$, $R^2$, $A^1$, $A^2$, $A^3$, $Z^1$ und $Z^2$ die angegebene Bedeutung, sofern nicht ausdrücklich etwas anderes vermerkt ist.

Die Verbindungen der Formel I umfassen dementsprechend Ic, If, Ii und Il (mit drei Ringen):

$R^1—A^1—A^2—A^3—R^2$ Ic

$R^1—A^1—Z^1—A^2—A^3—R^2$ If

$R^1—A^1—A^2—Z^2—A^3—R^2$ Ii

$R^1—A^1—Z^1—A^2—Z^2—A^3—R^2$ Il

Die bevorzugten Verbindungen der Teilformel Ic umfassen solche der Teilformeln Ica bis Iccc:

| | |
|---|---|
| $R^1$-Cy-Pyr-Phe-$R^2$ | Ica |
| $R^1$-Cy-Phe-Pyr-$R^2$ | Icc |
| $R^1$-Cy-Pyr-Cy-$R^2$ | Ice |
| $R^1$-Cy-Cy-Pyr-$R^2$ | Icf |
| $R^1$-Cy-Pyr-Dio-$R^2$ | Icg |
| $R^1$-Dio-Pyr-Cy-$R^2$ | Ich |
| $R^1$-Cy-Dio-Pyr-$R^2$ | Ici |
| $R^1$-Dio-Cy-Pyr-$R^2$ | Icj |
| $R^1$-Cy-Pyr-Dit-$R^2$ | Ick |
| $R^1$-Dit-Pyr-Cy-$R^2$ | Icl |
| $R^1$-Cy-Dit-Pyr-$R^2$ | Icm |
| $R^1$-Dit-Cy-Pyr-$R^2$ | Icn |
| $R^1$-Cy-Pyr-Bi-$R^2$ | Ico |
| $R^1$-Bi-Pyr-Cy-$R^2$ | Icp |
| $R^1$-Cy-Bi-Pyr-$R^2$ | Icq |
| $R^1$-Bi-Cy-Pyr-$R^2$ | Icr |
| $R^1$-Dio-Pyr-Phe-$R^2$ | Ics |
| $R^1$-Dio-Phe-Pyr-$R^2$ | Icu |
| $R^1$-Dit-Pyr-Phe-$R^2$ | Icw |
| $R^1$-Dit-Phe-Pyr-$R^2$ | Icy |
| $R^1$-Bi-Pyr-Phe-$R^2$ | Icaa |
| $R^1$-Bi-Phe-Pyr-$R^2$ | Iccc |

Darunter sind diejenigen der Teilformel Icf sowie Verbindungen der Teilformel Icc, worin $R^2$ CN bedeutet, besonders bevorzugt.

Die bevorzugten Verbindungen der Teilformel If umfassen solche der Teilformeln Ifc bis Ift:

| | |
|---|---|
| $R^1$-$A^1$-$CH_2CH_2$-$A^2$-$A^3$-$R^2$ | Ifc |
| $R^1$-$A^1$-$OCH_2$-$A^2$-$A^3$-$R^2$ | Ifd |
| $R^1$-$A^1$-$CH_2O$-$A^2$-$A^3$-$R^2$ | Ife |
| $R^1$-Cy-$Z^1$-$A^2$-$A^3$-$R^2$ | Iff |
| $R^1$-Dio-$Z^1$-$A^2$-$A^3$-$R^2$ | Ifg |
| $R^1$-Dit-$Z^1$-$A^2$-$A^3$-$R^2$ | Ifh |
| $R^1$-Bi-$Z^1$-$A^2$-$A^3$-$R^2$ | Ifi |
| $R^1$-Cy-$Z^1$-Pyr-Phe-$R^2$ | Ifj |
| $R^1$-Cy-$Z^1$-Phe-Pyr-$R^2$ | Ifk |
| $R^1$-Cy-$Z^1$-Pyr-Cy-$R^2$ | Ifl |
| $R^1$-Cy-$Z^1$-Cy-Pyr-$R^2$ | Ifm |

$R^1$-Cy-$Z^1$-Pyr-Dio-$R^2$ Ifn
$R^1$-Dio-$Z^1$-Pyr-Phe-$R^2$ Ifo
$R^1$-Dio-$Z^1$-Phe-Pyr-$R^2$ Ifp
$R^1$-Dit-$Z^1$-Pyr-Phe-$R^2$ Ifq
$R^1$-Dit-$Z^1$-Phe-Pyr-$R^2$ Ifr
$R^1$-Bi-$Z^1$-Pyr-Phe-$R^2$ Ifs
$R^1$-Bi-$Z^1$-Phe-Pyr-$R^2$ Ift

Darunter sind diejenigen der Teilformeln Ifc und Ife, sowie Ij, Ik und Il, besonders bevorzugt.
Die bevorzugten Verbindungen der Teilformel Ii umfassen solche der Teilformeln Iic bis Iiu:

$R^1$-$A^1$-$A^2$-$CH_2CH_2$-$A^3$-$R^2$ Iic
$R^1$-$A^1$-$A^2$-$CH_2O$-$A^3$-$R^2$ Iid
$R^1$-$A^1$-$A^2$-$OCH_2$-$A^3$-$R^2$ Iie
$R^1$-Cy-$A^2$-$Z^2$-$A^3$-$R^2$ Iif
$R^1$-Dio-$A^2$-$Z^2$-$A^3$-$R^2$ Iig
$R^1$-Dit-$A^2$-$Z^2$-$A^3$-$R^2$ Iih
$R^1$-Bi-$A^2$-$Z^2$-$A^3$-$R^2$ Iii
$R^1$-Cy-Pyr-$Z^2$-Phe-$R^2$ Iij
$R^1$-Cy-Pyr-$Z^2$-Cy-$R^2$ Iik
$R^1$-Cy-Phe-$Z^2$-Pyr-$R^2$ Iil
$R^1$-Cy-Cy-$Z^2$-Pyr-$R^2$ Iim
$R^1$-Dio-Cy-$Z^2$-Pyr-$R^2$ Iin
$R^1$-Dit-Cy-$Z^2$-Pyr-$R^2$ Iio
$R^1$-Dio-Pyr-$Z^2$-Phe-$R^2$ Iip
$R^1$-Dit-Pyr-$Z^2$-Phe-$R^2$ Iiq
$R^1$-Dio-Pyr-$Z^2$-Cy-$R^2$ Iir
$R^1$-Dit-Pyr-$Z^2$-Cy-$R^2$ Iis
$R^1$-Bi-Pyr-$Z^2$-Cy-$R^2$ Iit
$R^1$-Bi-Pyr-$Z^2$-Phe-$R^2$ Iin

Darunter sind diejenigen der Teilformel Iic besonders bevorzugt.
Die bevorzugten Verbindungen der Teilformel Il umfassen solche der Teilformeln Ili:

$R^1$-$A^1$-$CH_2CH_2$-$A^2$-$CH_2CH_2$-$A^3$-$R^2$ Ili

In den Verbindungen der vor- und nachstehenden Formeln bedeuten $R^1$ und $R^2$ vorzugsweise Alkyl, ferner Alkoxy, Oxaalkyl oder CN.

$A^1$ ist bevorzugt Cy oder Dio, ferner bevorzugt Dit oder Bi. Bevorzugt enthält die Verbindung der Formel I nicht mehr als einen der Reste Dio, Dit oder Bi.

$A^2$ und $A^3$ sind bevorzugt Phe oder Pyr, ferner bevorzugt Cy, Dio oder Dit.

$Z^1$ und $Z^2$ sind bevorzugt Einfachbindungen, in zweiter Linie bevorzugt —$CH_2CH_2$-Gruppen. Besonders bevorzugt sind Verbindungen der Formel I, worin eine der Gruppen $Z^1$ und $Z^2$ eine Einfachbindung, und die andere —$CH_2CH_2$—, —$OCH_2$— oder —$CH_2O$— bedeutet.

Die Alkylreste in den Gruppen $R^1$ und/oder $R^2$ können geradkettig oder verzweigt sein. Vorzugsweise sind sie geradkettig, haben 2, 3, 4, 5, 6, 7, 8, 9, 10, 11 oder 12 C-Atome und bedeuten demnach bevorzugt Ethyl, Propyl, Butyl, Pentyl, Hexyl, Heptyl, Octyl, Nonyl, Decyl, Undecyl oder Dodecyl, ferner Methyl, Tridecyl, Tetradecyl oder Pentadecyl.

Falls $R^1$ und/oder $R^2$ Alkylreste bedeuten, in denen eine ("Alkoxy" bzw. "Oxaalkyl") oder zwei ("Alkoxyalkoxy" bzw. "Dioxaalkyl") $CH_2$-Gruppen durch O-Atome ersetzt sind, so können sie geradkettig oder verzweigt sein. Vorzugsweise sind die geradkettig, haben 2, 3, 4, 5, 6 oder 7 C-Atome und bedueten demnach bevorzugt Ethoxy, Propoxy, Butoxy, Pentoxy, Hexoxy, Heptoxy, 2-Oxapropyl (= Methoxymethyl), 2- (=Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formeln I sowie der vor- und nachstehenden Teilformeln mit verzweigten Flügelgruppen $R^1$ bzw. $R^2$ können gelegentlich wegen einer besseren Löslichkeit in den üblichen flüssigkristallinen Basis-materialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Octyloxy.

Unter den Verbindungen der Formeln I sowie der vor- und nachstehenden Teilformeln sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln II bis IA sowie IB bis I20.

| | |
|---|---|
| Alkyl-Cy-Cy-Pyr-Alkyl | I1 |
| Alkyl-Cy-Cy-Pyr-Alkoxy | I2 |
| Alkyl-Dio-Cy-Pyr-Alkyl | I3 |
| Alkyl-Cy-$CH_2CH_2$-Phe-Pyr-Alkyl | I4 |
| Alkyl-Cy-$CH_2CH_2$-Phe-Pyr-Alkoxy | I5 |
| Alkyl-Cy-$CH_2CH_2$-Phe-Pyr-CN | I6 |
| Alkyl-Cy-Pyr-Phe-Alkoxy | I7 |
| Alkyl-Cy-$CH_2CH_2$-Pyr-Phe-Alkyl | I13 |
| Alkyl-Cy-$CH_2CH_2$-Pyr-Phe-Alkoxy | I14 |
| Alkyl-Cy-$CH_2CH_2$-Pyr-Phe-CN | I15 |
| Alkyl-Cy-Pyr-$CH_2CH_2$-Phe-Alkyl | I16 |
| Alkyl-Cy-Pyr-$CH_2CH_2$-Phe-Alkoxy | I17 |
| Alkyl-Cy-Pyr-$CH_2CH_2$-Phe-CN | I18 |
| Alkyl-Cy-Pyr-$CH_2CH_2$-Cy-Alkyl | I19 |
| Alkyl-Cy-$CH_2$-O-Phe-Pyr-Alkyl | I20 |

Darunter sind diejenigen der Formeln II, I2, I4, I5, I6, I13, I14, I15 und I20 besonders bevorzugt.

In den vorstehenden Formeln I 1 bis I 20 bedeutet Alkyl vorzugsweise eine geradkettige Alkylgruppe mit 2 bis 10 C-Atomen und Alkoxy eine geradkettige Alkoxygruppe mit 2 bis 12 C-Atomen. Weiterhin bevorzugt sind Verbindungen der Formeln I 1 bis I 20, in denen an Stelle von Alkoxy eine 2-Oxaalkylgruppe mit 2 bis 12 C-Atomen vorhanden ist.

Unter den Verbindungen der Formel I sind diejenigen Stereoisomeren bevorzugt, in denen die gesättigten Ringe (z.B. Cy, Dio, Dit) trans-1,4-disubstituiert sind.

Diejenigen der vorstehend genannten Formeln, die eine oder mehrere der Gruppen Dio, Dit und/oder Pyr enthalten, umschließen jeweils die beiden möglichen 2,5-Stellungsisomeren. Diejenigen der vorstehend genannten Formeln, in denen eine von der Einfachbindung verschiedene Gruppe $Z^1$ und/oder $Z^2$ mit Pyr in 5-Stellung verknüpft ist, sind bevorzugt.

Besonders bevorzugt sind Verbindungen der Formel I sowie der vorstehenden Teilformeln, worin $R^1$ eine geradkettige Alkylgruppe mit 1 bis 10 C-Atomen und $R^2$ eine geradkettige Alkoxy-, Oxaalkyl- oder Alkylgruppe mit 2 bis 15 C-Atomen, insbesondere mit 5 bis 12 C-Atomen beduetet.

Weiterhin bevorzugt sind verbindungen der Formel I worin $Z^1$ $-CH_2CH_2-$, $-OCH_2-$ oder $-CH_2O-$ bedeutet, falls $A^1$ 1,4-Cyclohexylen und

$-A^2-[Z^2-A^3]_n-$ 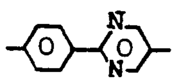 oder 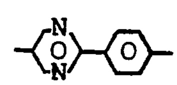 ist.

Die Verbindungen der Formel I werden nach an sich bekannten Methoden hergestellt. wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Der fachmann kann durch Routinemethoden entsprechende Synthesemethoden aus dem Stand der Technik entnehmen (z.B. DE—OS 23 44 732, 24 50 088, 24 29 093, 25 02 904, 26 36 684, 27 01 591 und 27 52 975 betreffend Verbindungen mit 1,4-Cyclohexylen- und 1,4-Phenylen-Gruppen; DE—PS 26 41 724 betreffend Verbindungen mit Pyrimidin-2,5-diyl-Gruppen; DE—OS 29 44 905 und 32 27 916 betreffend Verbindungen mit 1,3-Dioxan-2,5-diyl-Gruppen; DD 160 061 betreffend Verbindungen mit 1,3-Dithian-2,5-diyl-Gruppen, US 4,261,652 und 4,219,256 betreffend Verbindungen mit 1,4-Bicyclo(2,2,2)-octylen-Gruppen; und DE—OS 32 01 721 betreffend Verbindungen mit $-CH_2CH_2$-Brückengliedern.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man einer Verbindung, die sonst der Formel I entspricht, aber an Stelle von H-Atomen einer oder mehrere reduzierbare Gruppen und/oder C—C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer $-CH_2CH_2$-Gruppe eine $-CH{=}CH$-Gruppe und/oder an Stelle einer $-CH_2$-Gruppe eine —CO-Gruppe und/oder an Stelle eines H-Atomes eine freie oder eine funktionell (Z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropranol, einem Ether wie Tetrahydrofuran (THF) oder Dioxan, einem Ester wie Ethylacetate, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. $PtO_2$, PdO), auf einem Träger (z.B. Pd auf Kohle, Calciumcarbonat oder Strontriumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder —$CH_2CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden; so entstehen z.B. aus 1-Cyancyclohexenderivaten die entsprechenden Cyclohexanderivate.

Ester der Formel I ($R^1$ und/oder $R^2$ = Alkyl, worin eine oder zwei $CH_2$-Gruppen durch —O—CO— und/oder —CO—O-Gruppen ersetzt sind oder $Z^1$ und/oder $Z^2$ = —CO—O— oder —O—CO—) können auch durch Veresterung entsprechender Carbonsauren (oder ihren reaktionsfähigen Derivaten) mit Alkoholen bzw. Phenolen (oder ihrer reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1—4 C-Atomen in der Alkylgruppe.

Als reaktionsfähige Derivate der genannten Alkohole bzw. Phenole kommen insbesondere die entsprechenden Metallalkoholate bzw.-Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen −50° und +250°, vorzugsweise zwischen −20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Verestgerung besteht darin, daß man den Alkohol bzw. das Phenol zunächst in das Natrium- oder Kaliumalkoholat bzw. -phenolat überführt, z.B. durch gehandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa −25° und +20°.

Dioxanderivate bzw. Dithianderivate der Formel I (worin eine der Gruppen $A^1$ und/oder $A^2$ und/oder $A^3$ eine 1,3-Dioxan-2,5-diyl-Gruppe bzw. eine 1,3-Dithian-2,5-diyl-gruppe bedeutet) werden zweckmäßig durch Reaktion eines entsprechenden Aldehyds mit einem entsprechenden 1,3-Diol bzw. einem entsprechenden 1,3-Dithiol (oder einem seiner reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20°C und etwa 150°, vorzugsweise zwischen 80° und 120°. Als reaktionsfähige Derivate der Ausgangsstoffe eignen sich in erster Linie Acetale.

Die genannten Aldehyde, 1,3-Diole und 1,3-Dithiole sowie ihre reaktionsfähigen Derivate sind zum Teil

bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Carbonsäuren oder ihrere Derivate, die Diole durch Reduktion entsprechender Diester, sowie die Dithiole durch Umsetzung entsprechender Dihalogenide mit NaSH erhältlich.

Zur Herstellung von Nitrilen der Formel I (worin $R^1$ und/oder $R^2$ CN bedeuten) können entsprechende Säureamide dehydratisiert werden, Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z.B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I (worin $R^1$ und/oder $R^2$ eine Alkylgruppe bedeuten, worin eine oder zwei $CH_2$-Gruppen durch O-Atome ersetzt sind, und/oder worin $Z^1$ und/oder $Z^2$ eine $-OCH_2-$ oder eine $-CH_2O$-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Eine Base der Formel I kann mit einer Säure in das zugehörige Säureadditionssalz übergeführt werden. Für diese Umsetzung können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z.B. mit einer starken anorganischen Base wie KOH oder NaOH.

Die erfindungsgemäßen flüssigkristallinen Phasen bestehen aus 2 bis 15, vorzugsweise 3 bis 12 Komponenten, darunter mindestens einer Verbindung der formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder -cyclohexyl-ester, Phenylcyclohexane, Cyclohexylbiphenyl, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1,2-Bis-cyclohexylethane, 1,2-Bis-phenylethane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

$$R'-L-G-E-R'' \qquad II$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | —CH=CH— | —N(O)=N— |
| | —CH=CY— | —CH=N(O)— |
| | —C≡C— | $—CH_2—CH_2—$ |
| | —CO—O— | $—CH_2—O—$ |
| | —CO—S— | $—CH_2—S—$ |
| | —CH=N— | —COO—Phe—COO— |

oder eine C—C-Einfachbindung, Y Halogen, vorzugsweise Chlor, oder —CN, und R' und R'' Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind R' und R'' voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältich. Alle dieser Substanzen sind nach literaturbekannten Methoden herstellbar.

Die erfindungsgemäßen Phasen enthalten etwa 0,1 bis 99, vorzugsweise 10 bis 95%, einer oder mehrerer Verbindungen der Formel I.

Weiterhin bevorzugt sind erfindungsgemäße Dielektrika enthaltend 0,1 bis 40, vorzugsweise 0,5 bis 30% einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol. Cryst. Liq. Cryst. Band *24*, Seiten 249—258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE—OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturangaben sind in Grad Celsius angegeben. "Ubliche Aufarbeitung" bedeutet: man gibt wasser hinzu, extrahiert mit Methylenchlorid, trennt ab, trocknet die organische Phase, dampft ein und reinigt das Produkt durch Kristallisation und/oder Chromatographie.

### Beispiel 1

65 g des bekannten trans, trans-4'-Butyl-bicyclohexyl-4-carbonitrils werden in einem Gemisch aus 50 ml Ethanol und 50 ml Toluol gelöst. In die bei 10° gehaltene Lösung werden innerhalb von 2 h 27 g HCl geleitet, hierauf wird 16 h bei Raumtemperatur gerührt, der entstandene Niederschlag abgesaugt und mit Diethylether gewaschen. Ausbeute: 78 g trans, trans-4'-Butyl-bicyclohexyl-4'-carboximidsäureethylester-Hydrochlorid.

77 g des Imidesters werden in 140 ml Ethanol suspendiert und innerhalb von 15 Min bei Raumtemperatur zu einer Lösung von 22 g Ammoniak in 160 ml Ethanol gegeben. Nach 16 h wird das Lösungsmittel unter vermindertem Druck abdestilliert. Der Rückstand wird abgesaugt und mit Diethylether gewaschen. Ausbeute: 67 g trans, trans-4'-Butyl-bicyclohexyl-4-carboximidamid-Hydrochlorid.

60 g des Carboximidamids werden zusammen mit 40 g Pentylmalondialdehyd-bisdiethylacetal 15 h auf 150° erhitzt. Nach dem Abkühlen wird der Rückstand in Ethanol gelöst. Übliche Aufarbeitung liefert 2-(trans, trans-4'-Butyl-bicyclohexyl-4)-5-pentyl-pyrimidin.

Analog werden hergestellt:

2-(trans,trans-4'-Butyl-bicyclohexyl-4)-5-ethylpyrimidin
2-(trans,trans-4'-Butyl-bicyclohexyl-4)-5-propylpyrimidin
2-(trans,trans-4'-Butyl-bicyclohexyl-4)-5-butylpyrimidin
2-(trans,trans-4'-Butyl-bicyclohexyl-4)-5-heptylpyrimidin, F. 46°; S/N 137°; K. 147°; Δ ε 3,20
2-(trans,trans-4'-Ethyl-bicyclohexyl-4)-5-ethylpyrimidin
2-(trans,trans-4'-Ethyl-bicyclohexyl-4)-5-propylpyrimidin
2-(trans,trans-4'-Ethyl-bicyclohexyl-4)-5-butylpyrimidin
2-(trans,trans-4'-Ethyl-bicyclohexyl-4)-5-pentylpyrimidin
2-(trans,trans-4'-Ethyl-bicyclohexyl-4)-5-heptylpyrimidin

2-(trans,trans-4'-Ethyl-bicyclohexyl-4)-5-methoxypyrimidin
2-(trans,trans-4'-Ethyl-bicyclohexyl-4)-5-ethoxypyrimidin
2-(trans,trans-4'-Ethyl-bicyclohexyl-4)-5-propoxypyrimidin
2-(trans,trans-4'-Ethyl-bicyclohexyl-4)-5-butoxypyrimidin
2-(trans,trans-4'-Ethyl-bicyclohexyl-4)-5-pentoxypyrimidin
2-(trans,trans-4'-Ethyl-bicyclohexyl-4)-5-heptooxypyrimidin
2-(trans,trans-4'-Ethyl-bicyclohexyl-4)-5-cyanpyrimidin
2-(trans,trans-4'-Propyl-bicyclohexyl-4)-5-ethylpyrimidin
2-(trans,trans-4'-Propyl-bicyclohexyl-4)-5-propylpyrimidin
2-(trans,trans-4'-Propyl-bicyclohexyl-4)-5-butylpyrimidin
2-(trans,trans-4'-Propyl-bicyclohexyl-4)-5-pentylpyrimidin
2-(trans,trans-4'-Propyl-bicyclohexyl-4)-5-heptylpyrimidin
2-(trans,trans-4'-Propyl-bicyclohexyl-4)-5-methoxypyrimidin
2-(trans,trans-4'-Propyl-bicyclohexyl-4)-5-ethoxypyrimidin
2-(trans,trans-4'-Propyl-bicyclohexyl-4)-5-propoxypyrimidin
2-(trans,trans-4'-Propyl-bicyclohexyl-4)-5-butoxypyrimidin
2-(trans,trans-4'-Propyl-bicyclohexyl-4)-5-pentoxypyrimidin
2-(trans,trans-4'-Propyl-bicyclohexyl-4)-5-heptoxypyrimidin
2-(trans,trans-4'-Propyl-bicyclohexyl-4)-5-cyanpyrimidin
2-(trans,trans-4'-Pentyl-bicyclohexyl-4)-5-ethylpyrimidin
2-(trans,trans-4'-Pentyl-bicyclohexyl-4)-5-propylpyrimidin
2-(trans,trans-4'-Pentyl-bicyclohexyl-4)-5-butylpyrimidin
2-(trans,trans-4'-Pentyl-bicyclohexyl-4)-5-pentylpyrimidin
2-(trans,trans-4'-Pentyl-bicyclohexyl-4)-5-heptylpyrimidin
2-(trans,trans-4'-Pentyl-bicyclohexyl-4)-5-methoxypyrimidin
2-(trans,trans-4'-Pentyl-bicyclohexyl-4)-5-ethoxypyrimidin
2-(trans,trans-4'-Pentyl-bicyclohexyl-4)-5-propoxypyrimidin
2-(trans,trans-4'-Pentyl-bicyclohexyl-4)-5-butoxypyrimidin
2-(trans,trans-4'-Pentyl-bicyclohexyl-4)-5-pentoxypyrimidin
2-(trans,trans-4'-Pentyl-bicyclohexyl-4)-5-heptoxypyrimidin
2-(trans,trans-4'-Pentyl-bicyclohexyl-4)-5-cyanpyrimidin
2-(trans,trans-4'-Heptyl-bicyclohexyl-4)-5-ethylpyrimidin
2-(trans,trans-4'-Heptyl-bicyclohexyl-4)-5-propylpyrimidin
2-(trans,trans-4'-Heptyl-bicyclohexyl-4)-5-butylpyrimidin
2-(trans,trans-4'-Heptyl-bicyclohexyl-4)-5-pentylpyrimidin
2-(trans,trans-4'-Heptyl-bicyclohexyl-4)-5-heptylpyrimidin
2-(trans,trans-4'-Heptyl-bicyclohexyl-4)-5-methoxypyrimidin
2-(trans,trans-4'-Heptyl-bicyclohexyl-4)-5-ethoxypyrimidin
2-(trans,trans-4'-Heptyl-bicyclohexyl-4)-5-propoxypyrimidin
2-(trans,trans-4'-Heptyl-bicyclohexyl-4)-5-butoxypyrimidin
2-(trans,trans-4'-Heptyl-bicyclohexyl-4)-5-pentoxypyrimidin
2-(trans,trans-4'-Heptyl-bicyclohexyl-4)-5-heptoxypyrimidin
2-(trans,trans-4'-Heptyl-bicyclohexyl-4)-5-cyanpyrimidin

## Beispiel 2

Analog zu Beispiel 1 wird aus dem bekannten 4-(2-trans-4-Propylcyclohexyl-ethyl)-benzonitril über das entsprechende Carboximidsäureethylester-Hydrochlorid und das Carboximidamid-Hydrochlorid 2-[4-(2-trans-4-Propylcyclohexylethyl)-phenyl]-5-pentyl-pyrimidin hergestellt.

Analog werden hergestellt

2-[4-(2-trans-4-Propylcyclohexylethyl)-phenyl]-5-ethylpyrimidin
2-[4-(2-trans-4-Propylcyclohexylethyl)-phenyl]-5-propylpyrimidin
2-[4-(2-trans-4-Propylcyclohexylethyl)-phenyl]-5-butylpyrimidin
2-[4-(2-trans-4-Propylcyclohexylethyl)-phenyl]-5-heptylpyrimidin, F. 99°; K. 138°; $\Delta\varepsilon$ 1,90
2-[4-(2-trans-4-Propylcyclohexylethyl)-phenyl]-5-ethoxypyrimidin
2-[4-(2-trans-4-Propylcyclohexylethyl)-phenyl]-5-propoxypyrimidin
2-[4-(2-trans-4-Propylcyclohexylethyl)-phenyl]-5-pentyloxypyrimidin
2-[4-(2-trans-4-Propylcyclohexylethyl)-phenyl]-5-nonyloxypyrimidin
2-[4-(2-trans-4-Propylcyclohexylethyl)-phenyl]-5-cyanpyrimidin
2-[4-(2-trans-4-Ethylcyclohexylethyl)-phenyl]-5-ethylpyrimidin
2-[4-(2-trans-4-Ethylcyclohexylethyl)-phenyl]-5-propylpyrimidin
2-[4-(2-trans-4-Ethylcyclohexylethyl)-phenyl]-5-butylpyrimidin
2-[4-(2-trans-4-Ethylcyclohexylethyl)-phenyl]-5-pentylpyrimidin
2-[4-(2-trans-4-Ethylcyclohexylethyl)-phenyl]-5-heptylpyrimidin
2-[4-(2-trans-4-Ethylcyclohexylethyl)-phenyl]-5-nonylpyrimidin
2-[4-(2-trans-4-Ethylcyclohexylethyl)-phenyl]-5-ethoxypyrimidin

2-[4-(2-trans-4-Ethylcyclohexylethyl)-phenyl]-5-methoxypyrimidin
2-[4-(2-trans-4-Ethylcyclohexylethyl)-phenyl]-5-propoxypyrimidin
2-[4-(2-trans-4-Ethylcyclohexylethyl)-phenyl]-5-butoxypyrimidin
2-[4-(2-trans-4-Ethylcyclohexylethyl)-phenyl]-5-pentoxypyrimidin
2-[4-(2-trans-4-Ethylcyclohexylethyl)-phenyl]-5-hexoxypyrimidin
2-[4-(2-trans-4-Ethylcyclohexylethyl)-phenyl]-5-nonoxypyrimidin
2-[4-(2-trans-4-Ethylcyclohexylethyl)-phenyl]-5-cyanpyrimidin
2-[4-(2-trans-4-Butylcyclohexylethyl)-phenyl]-5-ethylpyrimidin
2-[4-(2-trans-4-Butylcyclohexylethyl)-phenyl]-5-propylpyrimidin
2-[4-(2-trans-4-Butylcyclohexylethyl)-phenyl]-5-butylpyrimidin
2-[4-(2-trans-4-Butylcyclohexylethyl)-phenyl]-5-pentylpyrimidin
2-[4-(2-trans-4-Butylcyclohexylethyl)-phenyl]-5-heptylpyrimidin
2-[4-(2-trans-4-Butylcyclohexylethyl)-phenyl]-5-nonylpyrimidin
2-[4-(2-trans-4-Butylcyclohexylethyl)-phenyl]-5-ethoxypyrimidin
2-[4-(2-trans-4-Butylcyclohexylethyl)-phenyl]-5-methoxypyrimidin
2-[4-(2-trans-4-Butylcyclohexylethyl)-phenyl]-5-propoxypyrimidin
2-[4-(2-trans-4-Butylcyclohexylethyl)-phenyl]-5-butoxypyrimidin
2-[4-(2-trans-4-Butylcyclohexylethyl)-phenyl]-5-pentoxypyrimidin
2-[4-(2-trans-4-Butylcyclohexylethyl)-phenyl]-5-hexoxypyrimidin
2-[4-(2-trans-4-Butylcyclohexylethyl)-phenyl]-5-nonoxypyrimidin
2-[4-(2-trans-4-Butylcyclohexylethyl)-phenyl]-5-cyanpyrimidin
2-[4-(2-trans-4-Pentylcyclohexylethyl)-phenyl]-5-ethylpyrimidin
2-[4-(2-trans-4-Pentylcyclohexylethyl)-phenyl]-5-propylpyrimidin
2-[4-(2-trans-4-Pentylcyclohexylethyl)-phenyl]-5-butylpyrimidin
2-[4-(2-trans-4-Pentylcyclohexylethyl)-phenyl]-5-pentylpyrimidin
2-[4-(2-trans-4-Pentylcyclohexylethyl)-phenyl]-5-heptylpyrimidin
2-[4-(2-trans-4-Pentylcyclohexylethyl)-phenyl]-5-nonylpyrimidin
2-[4-(2-trans-4-Pentylcyclohexylethyl)-phenyl]-5-ethoxypyrimidin
2-[4-(2-trans-4-Pentylcyclohexylethyl)-phenyl]-5-methoxypyrimidin
2-[4-(2-trans-4-Pentylcyclohexylethyl)-phenyl]-5-propoxypyrimidin
2-[4-(2-trans-4-Pentylcyclohexylethyl)-phenyl]-5-butoxypyrimidin
2-[4-(2-trans-4-Pentylcyclohexylethyl)-phenyl]-5-pentoxypyrimidin
2-[4-(2-trans-4-Pentylcyclohexylethyl)-phenyl]-5-hexoxypyrimidin
2-[4-(2-trans-4-Pentylcyclohexylethyl)-phenyl]-5-nonoxypyrimidin
2-[4-(2-trans-4-Pentylcyclohexylethyl)-phenyl]-5-cyanpyrimidin
2-[4-(2-trans-4-Heptylcyclohexylethyl)-phenyl]-5-ethylpyrimidin
2-[4-(2-trans-4-Heptylcyclohexylethyl)-phenyl]-5-propylpyrimidin
2-[4-(2-trans-4-Heptylcyclohexylethyl)-phenyl]-5-butylpyrimidin
2-[4-(2-trans-4-Heptylcyclohexylethyl)-phenyl]-5-pentylpyrimidin
2-[4-(2-trans-4-Heptylcyclohexylethyl)-phenyl]-5-heptylpyrimidin
2-[4-(2-trans-4-Heptylcyclohexylethyl)-phenyl]-5-nonylpyrimidin
2-[4-(2-trans-4-Heptylcyclohexylethyl)-phenyl]-5-ethoxypyrimidin
2-[4-(2-trans-4-Heptylcyclohexylethyl)-phenyl]-5-methoxypyrimidin
2-[4-(2-trans-4-Heptylcyclohexylethyl)-phenyl]-5-propoxypyrimidin
2-[4-(2-trans-4-Heptylcyclohexylethyl)-phenyl]-5-butoxypyrimidin
2-[4-(2-trans-4-Heptylcyclohexylethyl)-phenyl]-5-pentoxypyrimidin
2-[4-(2-trans-4-Heptylcyclohexylethyl)-phenyl]-5-hexoxypyrimidin
2-[4-(2-trans-4-Heptylcyclohexylethyl)-phenyl]-5-nonoxypyrimidin
2-[4-(2-trans-4-Heptylcyclohexylethyl)-phenyl]-5-cyanpyrimidin
2-p-Cyanphenyl-5-[2-(trans-4-nonylcyclohexyl)-ethyl]-pyrimidin
2-p-Fluorphenyl-5-[2-(trans-4-ethylcyclohexyl)-ethyl]-pyrimidin
2-p-Fluorphenyl-5-[2-(trans-4-propylcyclohexyl)-ethyl]-pyrimidin
2-p-Fluorphenyl-5-[2-(trans-4-butylcyclohexyl)-ethyl]-pyrimidin
2-p-Fluorphenyl-5-[2-(trans-4-pentylcyclohexyl)-ethyl]-pyrimidin
2-p-Fluorphenyl-5-[2-(trans-4-heptylcyclohexyl)-ethyl]-pyrimidin
2-p-Fluorphenyl-5-[2-(trans-4-nonylcyclohexyl)-ethyl]-pyrimidin
2-p-Ethylphenyl-5-[2-(trans-4-ethylcyclohexyl)-ethyl]-pyrimidin
2-p-Ethylphenyl-5-[2-(trans-4-propylcyclohexyl)-ethyl]-pyrimidin
2-p-Ethylphenyl-5-[2-(trans-4-butylcyclohexyl)-ethyl]-pyrimidin
2-p-Ethylphenyl-5-[2-(trans-4-pentylcyclohexyl)-ethyl]-pyrimidin
2-p-Ethylphenyl-5-[2-(trans-4-heptylcyclohexyl)-ethyl]-pyrimidin
2-p-Ethylphenyl-5-[2-(trans-4-nonylcyclohexyl)-ethyl]-pyrimidin
2-p-Butylphenyl-5-[2-(trans-4-ethylcyclohexyl)-ethyl]-pyrimidin
2-p-Butylphenyl-5-[2-(trans-4-propylcyclohexyl)-ethyl]-pyrimidin
2-p-Butylphenyl-5-[2-(trans-4-butylcyclohexyl)-ethyl]-pyrimidin

2-p-Butylphenyl-5-[2-(trans-4-pentylcyclohexyl)-ethyl]-pyrimidin
2-p-Butylphenyl-5-[2-(trans-4-heptylcyclohexyl)-ethyl]-pyrimidin
2-p-Butylphenyl-5-[2-(trans-4-nonylcyclohexyl)-ethyl]-pyrimidin
2-p-Pentylphenyl-5-[2-(trans-4-ethylcyclohexyl)-ethyl]-pyrimidin
2-p-Pentylphenyl-5-[2-(trans-4-propylcyclohexyl)-ethyl]-pyrimidin
2-p-Pentylphenyl-5-[2-(trans-4-butylcyclohexyl)-ethyl]-pyrimidin
2-p-Pentylphenyl-5-[2-(trans-4-pentylcyclohexyl)-ethyl]-pyrimidin
2-p-Pentylphenyl-5-[2-(trans-4-heptylcyclohexyl)-ethyl]-pyrimidin
2-p-Pentylphenyl-5-[2-(trans-4-nonylcyclohexyl)-ethyl]-pyrimidin
2-p-Heptylphenyl-5-[2-(trans-4-ethylcyclohexyl)-ethyl]-pyrimidin
2-p-Heptylphenyl-5-[2-(trans-4-propylcyclohexyl)-ethyl]-pyrimidin
2-p-Heptylphenyl-5-[2-(trans-4-butylcyclohexyl)-ethyl]-pyrimidin
2-p-Heptylphenyl-5-[2-(trans-4-pentylcyclohexyl)-ethyl]-pyrimidin
2-p-Heptylphenyl-5-[2-(trans-4-heptylcyclohexyl)-ethyl]-pyrimidin
2-p-Heptylphenyl-5-[2-(trans-4-nonylcyclohexyl)-ethyl]-pyrimidin
2-p-Hexylphenyl-5-[2-(trans-4-ethylcyclohexyl)-ethyl]-pyrimidin
2-p-Hexylphenyl-5-[2-(trans-4-propylcyclohexyl)-ethyl]-pyrimidin
2-p-Hexylphenyl-5-[2-(trans-4-butylcyclohexyl)-ethyl]-pyrimidin
2-p-Hexylphenyl-5-[2-(trans-4-pentylcyclohexyl)-ethyl]-pyrimidin
2-p-Hexylphenyl-5-[2-(trans-4-heptylcyclohexyl)-ethyl]-pyrimidin
2-p-Hexylphenyl-5-[2-(trans-4-nonylcyclohexyl)-ethyl]-pyrimidin
2-p-Nonylphenyl-5-[2-(trans-4-ethylcyclohexyl)-ethyl]-pyrimidin
2-p-Nonylphenyl-5-[2-(trans-4-propylcyclohexyl)-ethyl]-pyrimidin
2-p-Nonylphenyl-5-[2-(trans-4-butylcyclohexyl)-ethyl]-pyrimidin
2-p-Nonylphenyl-5-[2-(trans-4-pentylcyclohexyl)-ethyl]-pyrimidin
2-p-Nonylphenyl-5-[2-(trans-4-heptylcyclohexyl)-ethyl]-pyrimidin
2-p-Nonylphenyl-5-[2-(trans-4-nonylcyclohexyl)-ethyl]-pyrimidin
2-p-Methoxyphenyl-5-[2-(trans-4-ethylcyclohexyl)-ethyl]-pyrimidin
2-p-Methoxyphenyl-5-[2-(trans-4-propylcyclohexyl)-ethyl]-pyrimidin
2-p-Methoxyphenyl-5-[2-(trans-4-butylcyclohexyl)-ethyl]-pyrimidin
2-p-Methoxyphenyl-5-[2-(trans-4-pentylcyclohexyl)-ethyl]-pyrimidin
2-p-Methoxyphenyl-5-[2-(trans-4-heptylcyclohexyl)-ethyl]-pyrimidin
2-p-Methoxyphenyl-5-[2-(trans-4-nonylcyclohexyl)-ethyl]-pyrimidin
2-p-Pentoxyphenyl-5-[2-(trans-4-ethylcyclohexyl)-ethyl]-pyrimidin
2-p-Pentoxyphenyl-5-[2-(trans-4-propylcyclohexyl)-ethyl]-pyrimidin
2-p-Pentoxyphenyl-5-[2-(trans-4-butylcyclohexyl)-ethyl]-pyrimidin
2-p-Pentoxyphenyl-5-[2-(trans-4-pentylcyclohexyl)-ethyl]-pyrimidin
2-p-Pentoxyphenyl-5-[2-(trans-4-heptylcyclohexyl)-ethyl]-pyrimidin
2-p-Pentoxyphenyl-5-[2-(trans-4-nonylcyclohexyl)-ethyl]-pyrimidin
2-p-Hexoxyphenyl-5-[2-(trans-4-ethylcyclohexyl)-ethyl]-pyrimidin
2-p-Hexoxyphenyl-5-[2-(trans-4-propylcyclohexyl)-ethyl]-pyrimidin
2-p-Hexoxyphenyl-5-[2-(trans-4-butylcyclohexyl)-ethyl]-pyrimidin
2-p-Hexoxyphenyl-5-[2-(trans-4-pentylcyclohexyl)-ethyl]-pyrimidin
2-p-Hexoxyphenyl-5-[2-(trans-4-heptylcyclohexyl)-ethyl]-pyrimidin
2-p-Hexoxyphenyl-5-[2-(trans-4-nonylcyclohexyl)-ethyl]-pyrimidin
2-p-Heptoxyphenyl-5-[2-(trans-4-ethylcyclohexyl)-ethyl]-pyrimidin
2-p-Heptoxyphenyl-5-[2-(trans-4-propylcyclohexyl)-ethyl]-pyrimidin
2-p-Heptoxyphenyl-5-[2-(trans-4-butylcyclohexyl)-ethyl]-pyrimidin
2-p-Heptoxyphenyl-5-[2-(trans-4-pentylcyclohexyl)-ethyl]-pyrimidin
2-p-Heptoxyphenyl-5-[2-(trans-4-heptylcyclohexyl)-ethyl]-pyrimidin
2-p-Heptoxyphenyl-5-[2-(trans-4-nonylcyclohexyl)-ethyl]-pyrimidin
2-p-Octoxyphenyl-5-[2-(trans-4-ethylcyclohexyl)-ethyl]-pyrimidin
2-p-Octoxyphenyl-5-[2-(trans-4-propylcyclohexyl)-ethyl]-pyrimidin
2-p-Octoxyphenyl-5-[2-(trans-4-butylcyclohexyl)-ethyl]-pyrimidin
2-p-Octoxyphenyl-5-[2-(trans-4-pentylcyclohexyl)-ethyl]-pyrimidin
2-p-Octoxyphenyl-5-[2-(trans-4-heptylcyclohexyl)-ethyl]-pyrimidin
2-p-Octoxyphenyl-5-[2-(trans-4-nonylcyclohexyl)-ethyl]-pyrimidin
2-p-Nonoxyphenyl-5-[2-(trans-4-ethylcyclohexyl)-ethyl]-pyrimidin
2-p-Nonoxyphenyl-5-[2-(trans-4-propylcyclohexyl)-ethyl]-pyrimidin
2-p-Nonoxyphenyl-5-[2-(trans-4-butylcyclohexyl)-ethyl]-pyrimidin
2-p-Nonoxyphenyl-5-[2-(trans-4-pentylcyclohexyl)-ethyl]-pyrimidin
2-p-Nonoxyphenyl-5-[2-(trans-4-heptylcyclohexyl)-ethyl]-pyrimidin
2-p-Nonoxyphenyl-5-[2-(trans-4-nonylcyclohexyl)-ethyl]-pyrimidin
2-p-Decoxyphenyl-5-[2-(trans-4-ethylcyclohexyl)-ethyl]-pyrimidin
2-p-Decoxyphenyl-5-[2-(trans-4-propylcyclohexyl)-ethyl]-pyrimidin

2-p-Decoxyphenyl-5-[2-(trans-4-butylcyclohexyl)-ethyl]-pyrimidin
2-p-Decoxyphenyl-5-[2-(trans-4-pentylcyclohexyl)-ethyl]-pyrimidin
2-p-Decoxyphenyl-5-[2-(trans-4-heptylcyclohexyl)-ethyl]-pyrimidin
2-p-Decoxyphenyl-5-[2-(trans-4-nonylcyclohexyl)-ethyl]-pyrimidin
2-p-Undecoxyphenyl-5-[2-(trans-4-ethylcyclohexyl)-ethyl]-pyrimidin
2-p-Undecoxyphenyl-5-[2-(trans-4-propylcyclohexyl)-ethyl]-pyrimidin
2-p-Undecoxyphenyl-5-[2-(trans-4-butylcyclohexyl)-ethyl]-pyrimidin
2-p-Undecoxyphenyl-5-[2-(trans-4-pentylcyclohexyl)-ethyl]-pyrimidin
2-p-Undecoxyphenyl-5-[2-(trans-4-heptylcyclohexyl)-ethyl]-pyrimidin
2-p-Undecoxyphenyl-5-[2-(trans-4-nonylcyclohexyl)-ethyl]-pyrimidin
2-p-Dodecoxyphenyl-5-[2-(trans-4-ethylcyclohexyl)-ethyl]-pyrimidin
2-p-Dodecoxyphenyl-5-[2-(trans-4-propylcyclohexyl)-ethyl]-pyrimidin
2-p-Dodecoxyphenyl-5-[2-(trans-4-butylcyclohexyl)-ethyl]-pyrimidin
2-p-Dodecoxyphenyl-5-[2-(trans-4-pentylcyclohexyl)-ethyl]-pyrimidin
2-p-dodecoxyphenyl-5-[2-(trans-4-heptylcyclohexyl)-ethyl]-pyrimidin
2-p-Dodecoxyphenyl-5-[2-(trans-4-nonylcyclohexyl)-ethyl]-pyrimidin
2-(trans-4-Pentylcyclohexyl)-5-[2-(p-cyanphenyl)-ethyl]-pyrimidin
2-(trans-4-Pentylcyclohexyl)-5-[2-(p-methoxyphenyl)-ethyl]-pyrimidin
2-(trans-4-Pentylcyclohexyl)-5-[2-(p-propoxyphenyl)-ethyl]-pyrimidin
2-(trans-4-Pentylcyclohexyl)-5-[2-(p-pentoxyphenyl)-ethyl]-pyrimidin
2-(trans-4-Pentylcyclohexyl)-5-[2-(p-heptoxyphenyl)-ethyl]-pyrimidin
2-(trans-4-Pentylcyclohexyl)-5-[2-(p-nonoxyphenyl)-ethyl]-pyrimidin
2-(trans-4-Pentylcyclohexyl)-5-[2-(p-decoxyphenyl)-ethyl]-pyrimidin
2-(trans-4-Pentylcyclohexyl)-5-[2-(p-dodecoxyphenyl)-ethyl]-pyrimidin
2-(trans-4-Pentylcyclohexyl)-5-[2-(p-ethylphenyl)-ethyl]-pyrimidin
2-(trans-4-Pentylcyclohexyl)-5-[2-(p-propylphenyl)-ethyl]-pyrimidin
2-(trans-4-Pentylcyclohexyl)-5-[2-(p-butylphenyl)-ethyl]-pyrimidin
2-(trans-4-Pentylcyclohexyl)-5-[2-(p-pentylphenyl)-ethyl]-pyrimidin
2-(trans-4-Pentylcyclohexyl)-5-[2-(p-heptylphenyl)-ethyl]-pyrimidin
2-(trans-4-Pentylcyclohexyl)-5-[2-(p-nonylphenyl)-ethyl]-pyrimidin

Beispiel 3

Eine aus 6,9 g Natrium und 200 ml Ethanol hergestellte Natriumethanolat-Lösung wird nacheinander mit 28 g trans-4-Propylcyclohexyl-malonsäurediethylester und einer Aufschlämmung von 20 g 4-Ethoxybenzcarboxyimidamid-Hydrochlorid in 50 ml Ethanol versetzt. Nach stündigem Rühren wird das Reaktionsgemisch in 500 ml Eiswasser gegossen und mit verdünnter Salzsäure neutralisiert. Die Extraktion mit mehreren Portionen von Dichlormethan und die anschließende Abdestillation des Lösungsmittels ergibt einen Rückstand, der zweimal aus Ethanol umkristallisiert wird. Ausbeute: 23 g 4,6-Dihydroxy-2-(4-ethoxyphenyl)-5-(trans-4-propylcyclohexyl)-pyrimidin.

22 g des Dihydroxypyrimidins werden mit 19 g N,N-Diethylanilin und 80 ml Phosphoroxitrichlorid 48 h am Rückfluß erhitzt. Die abgekühlte Reaktionslösung wird auf Eis gegossen. Nach der Extraktion mit Dichlormethan und anschließender Abdestillation des Lösungsmittels wird der verbliebene Rückstand bestehend aus 4,6-Dichlor-2-(4-ethoxyphenyl)-5-(trans-4-propylcyclohexyl)-pyrimidin in 300 ml Methanol gelöst und nach Zusatz von Palladium-Kohle (5% Pd) bei Raumtemperatur hydriert. Der Katalysator wird durch Filtration entfernt, das Lösungsmittel abdestilliert und der Rückstand einer säulenchromatographischen Trennung unterzogen (Kieselgel/Toluol). Aus der Hauptfraktion wird nach Entfernen des Lösungsmittels und üblicher Aufarbeitung 2-(4-Ethoxyphenyl)-5-(trans-4-propylcyclohexyl)-pyrimidin erhalten.

Analog werden hergestellt:

2-(4-Ethoxyphenyl)-5-(trans-4-ethylcyclohexyl)-pyrimidin
2-(4-Ethoxyphenyl)-5-(trans-4-butylcyclohexyl)-pyrimidin
2-(4-Ethoxyphenyl)-5-(trans-4-pentylcyclohexyl)-pyrimidin
2-(4-Ethoxyphenyl)-5-(trans-4-heptylcyclohexyl)-pyrimidin
2-(4-Methoxyphenyl)-5-(trans-4-ethylcyclohexyl)-pyrimidin
2-(4-Methoxyphenyl)-5-(trans-4-propylcyclohexyl)-pyrimidin
2-(4-Methoxyphenyl)-5-(trans-4-butylcyclohexyl)-pyrimidin
2-(4-Methoxyphenyl)-5-(trans-4-pentylcyclohexyl)-pyrimidin
2-(4-Methoxyphenyl)-5-(trans-4-heptylcyclohexyl)-pyrimidin
2-(4-Propoxyphenyl)-5-(trans-4-ethylcyclohexyl)-pyrimidin
2-(4-Propoxyphenyl)-5-(trans-4-propylcyclohexyl)-pyrimidin
2-(4-Propoxyphenyl)-5-(trans-4-butylcyclohexyl)-pyrimidin
2-(4-Propoxyphenyl)-5-(trans-4-pentylcyclohexyl)-pyrimidin
2-(4-Propoxyphenyl)-5-(trans-4-heptylcyclohexyl)-pyrimidin
2-(4-Butoxyphenyl)-5-(trans-4-ethylcyclohexyl)-pyrimidin
2-(4-Butoxyphenyl)-5-(trans-4-propylcyclohexyl)-pyrimidin

2-(4-Butoxyphenyl)-5-(trans-4-butylcyclohexyl)-pyrimidin
2-(4-Butoxyphenyl)-5-(trans-4-pentylcyclohexyl)-pyrimidin
2-(4-Butoxyphenyl)-5-(trans-4-hexylcyclohexyl)-pyrimidin
2-(4-Butoxyphenyl)-5-(trans-4-heptylcyclohexyl)-pyrimidin
2-(4-Pentoxyphenyl)-5-(trans-4-ethylcyclohexyl)-pyrimidin
2-(4-Pentoxyphenyl)-5-(trans-4-propylcyclohexyl)-pyrimidin
2-(4-Pentoxyphenyl)-5-(trans-4-butylcyclohexyl)-pyrimidin
2-(4-Pentoxyphenyl)-5-(trans-4-pentylcyclohexyl)-pyrimidin
2-(4-Pentoxyphenyl)-5-(trans-4-heptylcyclohexyl)-pyrimidin
2-(4-Hexoxyphenyl)-5-(trans-4-ethylcyclohexyl)-pyrimidin
2-(4-Hexoxyphenyl)-5-(trans-4-propylcyclohexyl)-pyrimidin
2-(4-Hexoxyphenyl)-5-(trans-4-butylcyclohexyl)-pyrimidin
2-(4-Hexoxyphenyl)-5-(trans-4-pentylcyclohexyl)-pyrimidin
2-(4-Hexoxyphenyl)-5-(trans-4-heptylcyclohexyl)-pyrimidin
2-(4-Heptoxyphenyl)-5-(trans-4-ethylcyclohexyl)-pyrimidin
2-(4-Heptoxyphenyl)-5-(trans-4-propylcyclohexyl)-pyrimidin
2-(4-Heptoxyphenyl)-5-(trans-4-butylcyclohexyl)-pyrimidin
2-(4-Heptoxyphenyl)-5-(trans-4-pentylcyclohexyl)-pyrimidin
2-(4-Heptoxyphenyl)-5-(trans-4-heptylcyclohexyl)-pyrimidin
2-(4-Nonoxyphenyl)-5-(trans-4-ethylcyclohexyl)-pyrimidin
2-(4-Nonoxyphenyl)-5-(trans-4-propylcyclohexyl)-pyrimidin
2-(4-Nonoxyphenyl)-5-(trans-4-butylcyclohexyl)-pyrimidin
2-(4-Nonoxyphenyl)-5-(trans-4-pentylcyclohexyl)-pyrimidin
2-(4-Nonoxyphenyl)-5-(trans-4-heptylcyclohexyl)-pyrimidin
2-(4-Undecoxyphenyl)-5-(trans-4-ethylcyclohexyl)-pyrimidin
2-(4-Undecoxyphenyl)-5-(trans-4-propylcyclohexyl)-pyrimidin
2-(4-Undecoxyphenyl)-5-(trans-4-butylcyclohexyl)-pyrimidin
2-(4-Undecoxyphenyl)-5-(trans-4-pentylcyclohexyl)-pyrimidin
2-(4-Undecoxyphenyl)-5-(trans-4-heptylcyclohexyl)-pyrimidin
2-(4-Dodecoxyphenyl)-5-(trans-4-ethylcyclohexyl)-pyrimidin
2-(4-Dodecoxyphenyl)-5-(trans-4-propylcyclohexyl)-pyrimidin
2-(4-Dodecoxyphenyl)-5-(trans-4-butylcyclohexyl)-pyrimidin
2-(4-Dodecoxyphenyl)-5-(trans-4-pentylcyclohexyl)-pyrimidin
2-(4-Dodecoxyphenyl)-5-(trans-4-heptylcyclohexyl)-pyrimidin

## Beispiel 4

Eine Mischung von 11 g 2-p-Hydroxyphenyl-5-n-hexylpyrimidin, 7,8 g trans-4-n-Propyl-1-brommethylcyclohexan, 8,6 g Kaliumcarbonat und 50 ml Dimethylformamid wird 10 Stunden auf 90° erwärmt. Übliche Aufarbeitung liefert 4-(5-n-Hexylpyrimidin-2-yl)-phenyl-trans-4-n-propylcyclohexylmethyl-ether.

Analog werden hergestellt:

4-(5-Hexylpyrimidin-2-yl)-phenyl-trans-4-ethylcyclohexylmethyl-ether
4-(5-Hexylpyrimidin-2-yl)-phenyl-trans-4-butylcyclohexylmethyl-ether
4-(5-Hexylpyrimidin-2-yl)-phenyl-trans-4-pentylcyclohexylmethyl-ether, F. 99°, K. 150°
4-(5-Hexylpyrimidin-2-yl)-phenyl-trans-4-heptylcyclohexylmethyl-ether
4-(5-Heptylpyrimidin-2-yl)-phenyl-trans-4-ethylcyclohexylmethyl-ether
4-(5-Heptylpyrimidin-2-yl)-phenyl-trans-4-propylcyclohexylmethyl-ether
4-(5-Heptylpyrimidin-2-yl)-phenyl-trans-4-butylcyclohexylmethyl-ether
4-(5-Heptylpyrimidin-2-yl)-phenyl-trans-4-pentylcyclohexylmethyl-ether
4-(5-Heptylpyrimidin-2-yl)-phenyl-trans-4-heptylcyclohexylmethyl-ether
4-(5-Nonylpyrimidin-2-yl)-phenyl-trans-4-ethylcyclohexylmethyl-ether
4-(5-Nonylpyrimidin-2-yl)-phenyl-trans-4-propylcyclohexylmethyl-ether
4-(5-Nonylpyrimidin-2-yl)-phenyl-trans-4-butylcyclohexylmethyl-ether
4-(5-Nonylpyrimidin-2-yl)-phenyl-trans-4-pentylcyclohexylmethyl-ether
4-(5-Nonylpyrimidin-2-yl)-phenyl-trans-4-heptylcyclohexylmethyl-ether
4-(5-Pentylpyrimidin-2-yl)-phenyl-trans-4-ethylcyclohexylmethyl-ether
4-(5-Pentylpyrimidin-2-yl)-phenyl-trans-4-propylcyclohexylmethyl-ether
4-(5-Pentylpyrimidin-2-yl)-phenyl-trans-4-butylcyclohexylmethyl-ether
4-(5-Pentylpyrimidin-2-yl)-phenyl-trans-4-pentylcyclohexylmethyl-ether
4-(5-Pentylpyrimidin-2-yl)-phenyl-trans-4-heptylcyclohexylmethyl-ether
4-(5-Propylpyrimidin-2-yl)-phenyl-trans-4-ethylcyclohexylmethyl-ether
4-(5-Propylpyrimidin-2-yl)-phenyl-trans-4-propylcyclohexylmethyl-ether
4-(5-Propylpyrimidin-2-yl)-phenyl-trans-4-butylcyclohexylmethyl-ether
4-(5-Propylpyrimidin-2-yl)-phenyl-trans-4-pentylcyclohexylmethyl-ether
4-(5-Propylpyrimidin-2-yl)-phenyl-trans-4-heptylcyclohexylmethyl-ether

Die folgenden Beispiel betreffen erfindungsgemäße flüssigkristalline Phasen:

Beispiel A

Man stellt eine flüssigkristalline Phase her aus
17% p-trans-4-Propylcyclohexyl-benzonitril,
23% p-trans-4-Pentylcyclohexyl-benzonitril,
16% trans-1-p-Ethoxyphenyl-4-propylcyclohexan,
14% trans-1-p-Butoxyphenyl-4-propylcyclohexan,
10% 4-(trans-4-Pentylcyclohexyl)-4'-(trans-4-propylcyclohexyl)-biphenyl,
10% 2-(4-Ethoxyphenyl)-5-(trans-4-propylcyclohexyl)-pyrimidin und
10% 2-(4-Methoxyphenyl)-5-(trans-4-pentylcyclohexyl)-pyrimidin.

Beispiel B

Man stellt eine flüssigkristalline Phase her aus
21% p-trans-4-Ethylcyclohexyl-benzonitril,
22% p-trans-4-Butylcyclohexyl-benzonitril,
14% 4-Ethyl-4'-cyanbiphenyl,
18% 4-Butyl-4'-cyanbiphenyl,
10% 2-(trans-4-Propylcyclohexyl)-pyrimidin-5-carbonsäure-(p-pentylphenylester) und
15% 2-[trans-4-(trans-5-Propyl-1,3-dioxan-2-yl)-cyclohexyl]-5-pentylpyrimidin.

Beispiel C

Eine flüssigkristalline Phase aus
8% 2-p-Cyanphenyl-5-butyl-1,3-dioxan,
7% 2-p-Cyanphenyl-5-pentyl-1,3-dioxan,
8% 4-Cyan-4'-(trans-4-pentylcyclohexyl)-biphenyl,
5% 4-Ethyl-4'-(trans-4-pentylcyclohexyl)-biphenyl,
5% 2-(4-Ethoxyphenyl)-5-(trans-4-propylcyclohexyl)-pyrimidin,
5% 2-p-Pentoxyphenyl-5-hexylpyrimidin,
5% 2-p-Hexoxyphenyl-5-hexylpyrimidin,
6% 2-p-Heptoxyphenyl-5-hexylpyrimidin,
8% 2-p-Nonoxyphenyl-5-hexylpyrimidin,
8% 2-p-Undecoxyphenyl-5-hexylpyrimidin,
7% trans-4-Propylcyclohexancarbonsäure-(p-methoxyphenylester),
7% trans-4-Propylcyclohexancarbonsäure-(p-ethoxyphenylester),
7% trans-4-Butylcyclohexancarbonsäure-(p-methoxyphenylester),
7% trans-4-Butylcyclohexancarbonsäure-(p-ethoxyphenylester) und
7% trans-4-Pentylcyclohexancarbonsäure-(p-methoxyphenylester)
hat eine Viskosität von 49. $10^{-3}$ Pa.s und ist gut geeignet für hoch multiplexierbare Flüssigkristallanzeigeelemente.

Beispiel D

Eine flüssigkristalline Phase aus
8,0% trans-1-p-Propylphenyl-4-pentylcyclohexan,
7,0% 2-p-Cyanphenyl-5-propyl-1,3-dioxan,
8,0% 2-p-Cyanphenyl-5-butyl-1,3-dioxan,
7,0% 2-p-Cyanphenyl-5-pentyl-1,3-dioxan,
7,5% 4-Cyan-4'-(trans-4-pentylcyclohexyl)-biphenyl,
5,0% 4-Ethyl-4'-(trans-4-pentylcyclohexyl)-biphenyl,
5,0% 4-Ethyl-4'-(trans-4-propylcyclohexyl)-biphenyl,
5,0% 2-p-Pentoxyphenyl-5-hexylpyrimidin,
5,0% 2-p-Hexoxyphenyl-5-hexylpyrimidin,
6,0% 2-p-Heptoxyphenyl-5-hexylpyrimidin,
8,0% 2-p-Nonoxyphenyl-5-hexylpyrimidin,
8,0% 2-p-Undecoxyphenyl-5-hexylpyrimidin,
0,5% 2-(trans,trans-4'-Butyl-bicyclohexyl-4)-5-pentylpyrimidin,
7,0% trans-4-Propylcyclohexancarbonsäure-(p-methoxyphenylester),
7,0% trans-4-Propylcyclohexancarbonsäure-(p-ethoxyphenylester) und
6,0% trans-4-Butylcyclohexancarbonsäure-(p-methoxyphenylester)
hat einen Klärpunkt von 64°, eine Viskosität von $45.10^{-3}$ Pa.s und ist gut geeignet für hoch multiplexierbare Flüssigkristallanzeigeelemente.

Beispeil E

Ein flüssigkristalline Phase aus
7% 2-p-Cyanphenyl-5-propyl-1,3-dioxan,

6% 2-p-Cyanphenyl-5-butyl-1,3-dioxan,
5% 2-p-Cyanphenyl-5-pentyl-1,3-dioxan,
4% 2-p-Pentoxyphenyl-5-hexylpyrimidin,
4% 2-p-Hexoxyphenyl-5-hexylpyrimidin,
5% 2-p-Heptoxyphenyl-5-hexylpyrimidin,
7% 2-p-Nonoxyphenyl-5-hexylpyrimidin,
7% 2-p-Undecoxyphenyl-5-hexylpyrimidin,
6% trans-4-Propylcyclohexancarbonsäure-(p-methoxyphenylester),
4% trans-4-Butylcyclohexancarbonsäure-(p-methoxyphenylester),
3% trans-4-Pentylcyclohexancarbonsäure-(p-methoxyphenylester),
9% trans-1-p-Propylphenyl-4-pentylcyclohexan,
3% Buttersäure-(p-trans-4-propylcyclohexyl-phenylester),
18% 2-(trans,trans-4'-Butyl-bicyclohexyl-4)-5-pentylpyrimidin,
9% 2-[4-(2-trans-4-Propylcyclohexylethyl)-phenyl]-5-pentylpyrimidin und
3% 2-[4-(2-trans-4-Propylcyclohexylethyl)-phenyl]-5-heptylpyrimidin
ist gut geeignet für hoch multiplexierbare Flüssigkristallanzeigeelemente.

Beispiel F

Eine flüssigkristalline Phase aus
3% 2-p-Cyanphenyl-5-pentyl-1,3-dioxan,
6% 4-Cyan-4'-(trans-4-pentylcyclohexyl)-biphenyl,
7% 2-[4-(2-trans-4-Propylcyclohexylethyl)-phenyl]-5-pentylpyrimidin,
8% 2-[4-(2-trans-4-Propylcyclohexylethyl)-phenyl]-5-heptylpyrimidin,
5% 2-p-Octoxyphenyl-5-pentylpyrimidin,
5% 2-p-Pentoxyphenyl-5-hexylpyrimidin,
5% 2-p-Heptoxyphenyl-5-hexylpyrimidin,
4% 2-p-Nonoxyphenyl-5-hexylpyrimidin,
5% 2-p-Heptoxyphenyl-5-heptylpyrimidin,
4% 2-p-Nonoxyphenyl-5-heptylpyrimidin,
5% 2-p-Nonoxyphenyl-5-nonylpyrimidin,
5% trans-4-Propylcyclohexancarbonsäure-(p-methoxyphenylester),
7% trans-4-Butylcyclohexancarbonsäure-(p-methoxyphenylester),
8% trans-4-Pentylcyclohexancarbonsäure-(p-methoxyphenylester),
17% trans-1-p-Propylphenyl-4-pentylcyclohexan und
6% Buttersäure-(p-trans-4-propylcyclohexylphenylester)
hat einen Klärpunkt von 65°. Diese Mischung ist gut geeignet für hoch multiplexierbare Flüssigkristallanzeigeelemente.

**Patentansprüche für die Vertragsstaaten: CH DE GB LI**

1. Verbindungen der Formel I

$$R^1—A^1—Z^1—A^2—Z^2—A^3—R^2$$

worin

$R^1$ und $R^2$ jeweils unabhängig voneinander eine Alkylgruppe mit 1—15 C-Atomen, worin auch eine oder zwei nicht benachbarte $CH_2$-Gruppen durch O-Atome und/oder —CO-Gruppen und/oder —O—CO-Gruppen und/oder —CO—O-Gruppen ersetzt sein können, einer der Reste $R^1$ und $R^2$ auch H, F, Cl, Br oder CN,

$A^1$ eine 1,4-Cyclohexylengruppe, worin auch eine oder zwei nicht benachbarte $CH_2$-Gruppen durch O-Atome ersetzt sein können, eine 1,4-Bicyclo-(2,2,2)-octylengruppe oder eine 1,3-Dithian-2,5-diyl-Gruppe,

$Z^1$ und $Z^2$ jeweils unabhängig voneinander $—CH_2CH_2—$, $—OCH_2$, $—CH_2O—$ oder eine Einfachbindung, und

$A^2$ und $A^3$ jeweils unabhängig voneinander eine 1,4-Phenylengruppe, Pyrimidin-2,5-diyl-gruppe, 1,4-Cyclohexylen-gruppe, worin auch eine oder zwei nicht benachbarte $CH_2$-Gruppen durch O-Atome ersetzt sein können, 1,3-Dithian-2,5-diylgruppe oder eine 1,4-Bicyclo(2,2,2)-octylengruppe bedeuten,

mit den Maßgaben, daß

(a) mindestens eine der Gruppen $A^2$ und $A^3$ eine Pyrimidin-2,5-diylgruppe ist,

(b) mindestens eine der Gruppen $Z^1$ und $Z^2$ keine Einfachbindung bedeutet, wenn $A^2$ Pyrimidin-2,5-diyl und $R^2$ Alkyl oder CN ist,

(c) 2-(4-Cyanphenyl)-5-[2-(trans-4-ethylcyclohexyl)-ethyl]-pyrimidin,
2-(4-Cyanphenyl)-5-[2-(trans-4-propylcyclohexyl)-ethyl]-pyrimidin,
2-(4-Cyanphenyl)-5-[2-(trans-4-butylcyclohexyl)-ethyl]-pyrimidin,
2-(4-Cyanphenyl)-5-[2-(trans-4-pentylcyclohexyl)-ethyl]-pyrimidin und
2-(4-Cyanphenyl)-5-[2-(trans-4-heptylcyclohexyl)-ethyl]-pyrimidin,
ausgeschlossen ist, und

(d) 5-Alkyl-2-(4'-(trans-4''-alkylcyclohexyl)phenyl)pyrimidine ausgeschlossen sind.

2. Verbindungen nach Anspruch 1, worin $R^1$ und $R^2$ jeweils unabhängig voneinander eine geradkettige Alkyl-, Alkoxy- oder Oxalkylgruppe bedeutet.

3. Verbindungen nach Anspruch 1 oder 2, worin
$A^1$ eine trans-1,4-Cyclohexylengruppe bedeutet.

4. Verbindungen nach einem der Ansprüche 1 bis 3 entsprechend der Teilformeln Ica, Ifi, Ifk, Ifj und Ifl

| | |
|---|---|
| $R^1$-Cy-Pyr-Phe-$R^2$ | Ica |
| $R^1$-Cy-$Z^1$-Pyr-Phe-$R^2$ | Ifi |
| $R^1$-Cy-$Z^1$-Phe-Pyr-$R^2$ | Ifk |
| $R^1$-Cy-Pyr-$Z^2$-Phe-$R^2$ | Iij |
| $R^1$-Cy-Phe-$Z^2$-Pyr-$R^2$ | Iil, |

worin $R^1$ und $R^2$ die angegebene Bedeutung besitzen, und
Cy eine 1,4-Cyclohexylengruppe,
Pyr eine Pyrimidin-2,5-diylgruppe,
Phe eine 1,4-Phenylengruppe und
$Z^1$ und $Z^2$ $—CH_2O—$, $—OCH_2—$ oder $—CH_2CH_2—$ bedeuten.

5. Verbindungen nach Anspruch 4 entsprechend der Teilformeln Ica, Ifi, Ifk, Iij und Iil, worin $R^1$, $Z^1$, $Z^2$, Cy, Pyr und Phe die angegebene Bedeutung besitzen, und
$R^2$ F bedeutet.

6. Verbindungen nach Anspruch 4 oder 5 worin $Z^1$ bzw.
$Z^2$ $—CH_2CH_2—$ oder $—CH_2O—$ bedeutet.

7. Verbindungen nach Anspruch 3 entsprechend den Teilformeln
Alkyl-Cy-Cy-Pyr-Alkyl
Alkyl-Cy-Cy-Pyr-Alkoxy
worin Cy und Pyr die in Anspruch 2 angegebene Bedeutung besitzen, und Alkyl eine geradkettige alkylgruppe mit 2 bis 10 C-Atomen und Alkoxy eine geradkettige Alkoxygruppe mit 2 bis 12 C-Atomen bedeutet.

8. Verbindungen nach Anspruch 4 entsprechend der Teilformeln
Alkyl-Cy-$CH_2CH_2$-Phe-Pyr-Alkyl
Alkyl-Cy-$CH_2O$-Phe-Pyr-Alkyl
worin Cy, Phe und Pyr die in Anspruch 4 gegebene Bedeutung besitzen, und Alkyl jeweils eine geradkettige Alkylgruppe mit 2 bis 10 C-Atomen bedeutet.

9. Verbindungen nach Anspruch 4 entsprechend der Teilformeln
Alkyl-Cy-$CH_2CH_2$-Pyr-Phe-Alkyl
Alkyl-Cy-$CH_2CH_2$-Pyr-Phe-F
Alkyl-Cy-$CH_2CH_2$-Pyr-Phe-CN
worin Cy, Phe und Pyr die in Anspruch 4 gegebene Bedeutung besitzen, und Alkyl jeweils eine geradkettige Alkylgruppe mit 2 bis 10 C-Atomen bedeutet.

10. Verfahren zur Herstellung von Verbindungen der Formel I nach Anspruch 1, dadurch gekennzeichnet, daß man eine Verbindung, die sonst der Formel I entspricht, aber an Stelle von H-Atomen eine oder mehrere reduzierbare Gruppen und/oder C—C-Bindungen enthält, mit einem reduzierenden Mittel behandelt.
oder daß man zur Herstellung von Estern der Formel I (worin $R^1$ und/oder $R^2$ eine Alkylgruppe bedeuten, worin eine oder zwei $CH_2$-Gruppen durch —O—CO-Gruppen und/oder —CO—O-Gruppen ersetzt sind) eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von 1,3-Dioxanderivaten bzw. 1,3-Dithianderivaten der Formel I (worin $A^1$ 1,3-Dioxan-2,5-diyl bzw. 1,3-Dithian-2,5-diyl bedeutet) einen entsprechenden Aldehyd mit einem entsprechenden Diol bzw. Dithiol umsetzt,
oder daß man zur Herstellung von Ethern der Formel I (worin $R^1$ und/oder $R^2$ eine Alkylgruppe bedeuten, worin eine oder zwei $CH_2$-Gruppen durch O-Atome ersetzt sind und/oder Z ein $—OCH_2—$ oder $—CH_2O$-Gruppe ist) eine entsprechende Hydroxyverbindung verethert,

11. Verwendung der Verbindungen der Formel I nach Anspruch 1 als Komponenten flüssigkristalliner Phasen.

12. Flüssigkristalline Phase mit mindestens zwei flüssigkristallinen Komponenten, dadurch gekennzeichnet, daß mindestens eine Komponente eine Verbindung der Formel I nach Anspruch 1 ist.

13. Flüssigkristalline Phase mit mindestens zwei flüssigkristallinen Komponenten, dadurch gekennzeichnet, daß mindestens eine Komponente eine Verbindung der Formel I ist,

$$R^1—A^1—Z^1—A^2—Z^2—A^3—R^2 \qquad I$$

worin $R^1$, $R^2$, $A^1$, $A^2$, $A^3$, $Z^1$ und $Z^2$ die in Anspruch 1 angegebene Bedeutung besitzen mit den Maßgaben, daß

(a) mindestens eine der Gruppen $A^2$ und $A^3$ eine Pyrimidin-2,5-diylgruppe ist, und

(b) mindestens eine der Gruppen $Z^1$ und $Z^2$ keine Einfachbindung bedeutet, wenn $A^2$ Pyrimidin-2,5-diyl und $R^2$ Alkyl oder CN ist,

(c) 2-(4-Cyanphenyl)-5-[2-(trans-4-ethylcyclohexyl)-ethyl]pyrimidin,
2-(4-Cyanphenyl)-5-[2-(trans-4-propylcyclohexyl)-ethyl]-pyrimidin,
2-(4-Cyanphenyl)-5-[2-(trans-4-butylcyclohexyl)-ethyl]-pyrimidin,
2-(4-Cyanphenyl)-5-[2-(trans-4-pentylcyclohexyl)-ethyl]-pyrimidin und
2-(4-Cyanphenyl)-5-[2-(trans-4-heptylcyclohexyl)-ethyl]-pyrimidin,

ausgeschlossen ist, und sie mindestens einen Bestandteil der Formel II enthält

$$R'—L—G—E—R'' \qquad II$$

worin

L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinalozin gebildeten Gruppe,

| | | |
|---|---|---|
| G | —CH=CH— | —N(O)=N— |
| | —CH=CY— | —CH=N(O)— |
| | —C≡C— | $—CH_2—CH_2—$ |
| | —CO—O— | $—CH_2—O—$ |
| | —CO—S— | $—CH_2—S—$ |
| | —CH=N— | —COO—Ph—COO— |

Y Halogen oder —CH, und

R' und R'' Alkoxy, Alkanoyloxy oder Alkoxycarbonyloxy mit bis zu 8 Kohlenstoffatomen, oder einer dieser Reste auch CN, NC, $NO_2$, $CF_3$, F, Cl oder Br bedeuten, oder sie mindestens einen anderen Bestandteil enthält, ausgewählt aus den Klassen der Benzylidenaniline, Terphenyle, Cyclohexylbiphenyle, Cyclohexylcyclohexane, Cyclohexylnaphthaline, 1,4-Bis-cyclohexylbenzole, 4,4'-Bis-cyclohexylbiphenyle, gegebenenfalls halogenierten Stilbene, Benzylphenylether, Tolane und substituierten Zimtsäuren.

14. Flüssigkristallanzeigeelement, dadurch gekennzeichnet, daß es eine Phase nach mindestens einem der Ansprüche 12 und 13 enthält.

15. Elektrooptisches Anzeigeelment nach Anspruch 14, dadurch gekennzeichnet, daß es als Dielektrikum eine Phase nach mindestens einen der Ansprüche 12 und 13 enthält.

**Patentansprüche für den Vertragsstaat: FR**

1. Verbindungen der Formel I

$$R^1—A^1—Z^1—A^2—Z^2—A^3—R^2$$

worin

$R^1$ und $R^2$ jeweils unabhängig voneinander eine Alkylgruppe mit 1—15 C-Atomen, worin auch eine oder zwei nicht benachbarte $CH_2$-Gruppen durch O-Atome und/oder —CO-Gruppen und/oder —O—CO-Gruppen und/oder —CO—O-Gruppen ersetzt sein könne, einer der Reste $R^1$ und $R^2$ auch H, F, Cl oder Br,

$A^1$ eine 1,4-Cyclohexylengruppe, worin auch eine oder zwei nicht benachbarte $CH_2$-Gruppen durch O-Atome ersetzt sein können, eine 1,4-Bicyclo-(2,2,2)-octylengruppe oder eine 1,3-Dithian-2,5-diyl-Gruppe,

$Z^1$ und $Z^2$ jeweils unabhängig voneinander $—CH_2CH_2—$, $—OCH_2$, $—CH_2O—$ oder eine Einfachbindung, und

$A^2$ und $A^3$ jeweils unabhängig voneinander eine 1,4-Phenylengruppe, Pyrimidin-2,5-diyl-gruppe, 1,4-Cyclohexylen-gruppe, worin auch eine oder zwei nicht benachbarte $CH_2$-Gruppen durch O-Atome ersetzt sein können, 1,3-Dithian-2,5-diylgruppe oder eine 1,4-Bicyclo(2,2,2)-octylengruppe bedeuten,

mit den Maßgaben, daß

(a) mindestens eine der Gruppen $A^2$ und $A^3$ eine Pyrimidin-2,5-diylgruppe ist,

(b) mindestens eine der Gruppen $Z^1$ und $Z^2$ keine Einfachbindung bedeutet, wenn $A^2$ Pyrimidin-2,5-diyl und $R^2$ Alkyl oder CN ist,

(c) 2-(4-Cyanphenyl)-5-[2-(trans-4-ethylcyclohexyl)-ethyl]-pyrimidin,
2-(4-Cyanphenyl)-5-[2-(trans-4-propylcyclohexyl)-ethyl]-pyrimidin,
2-(4-Cyanphenyl)-5-[2-(trans-4-butylcyclohexyl)-ethyl]-pyrimidin,

2-(4-Cyanphenyl)-5-[2-(trans-4-pentylcyclohexyl)-ethyl]-pyrimidin und
2-(4-Cyanphenyl)-5-[2-(trans-4-heptylcyclohexyl)-ethyl]-pyrimidin,
ausgeschlossen ist,

2. Verbindungen nach Anspruch 1, worin $R^1$ und $R^2$ jeweils unabhängig voneinander eine geradkettige Alkyl-, Alkoxy- oder Oxaalkylgruppe bedeutet.

3. Verbindungen nach Anspruch 1 oder 2, worin
$A^1$ eine trans-1,4-Cyclohexylengruppe bedeutet.

4. Verbindungen nach einem der Ansprüche 1 bis 3 entsprechend der Teilformeln Ica, Ifi, Ifk, Ifj und Ifl

| | |
|---|---|
| $R^1$-Cy-Pyr-Phe-$R^2$ | Ica |
| $R^1$-Cy-$Z^1$-Pyr-Phe-$R^2$ | Ifi |
| $R^1$-Cy-$Z^1$-Phe-Pyr-$R^2$ | Ifk |
| $R^1$-Cy-Pyr-$Z^2$-Phe-$R^2$ | Iij |
| $R^1$-Cy-Phe-$Z^2$-Pyr-$R^2$ | Iil, |

worin $R^1$ und $R^2$ die angegebene Bedeutung besitzen, und
Cy eine 1,4-Cyclohexylengruppe,
Pyr eine Pyrimidin-2,5-diylgruppe,
Phe eine 1,4-Phenylengruppe und
$Z^1$ und $Z^2$ —$CH_2O$—, —$OCH_2$— oder —$CH_2CH_2$— bedeuten.

5. Verbindungen nach Anspruch 4 entsprechend der Teilformeln Ica, Ifi, Ifk, Iij und Iil, worin $R^1$, $Z^1$, $Z^2$, Cy, Pyr und Phe die angegebene Bedeutung besitzen, und
$R^2$ F bedeutet.

6. Verbindungen nach Anspruch 4 oder 5 worin $Z^1$ bzw. $Z^2$ —$CH_2CH_2$— oder —$CH_2O$— bedeutet.

7. Verbindungen nach Anspruch 3 entsprechend den Teilformeln
Alkyl-Cy-Cy-Pyr-Alkyl
Alkyl-Cy-Cy-Pyr-Alkoxy
worin Cy und Pyr die in Anspruch 2 angegebene Bedeutung besitzen, und Alkyl eine geradkettige Alkylgruppe mit 2 bis 10 C-Atomen und Alkoxy eine geradkettige Alkoxygruppe mit 2 bis 12 C-Atomen bedeutet.

8. Verbindungen nach Anspruch 4 entsprechend der Teilformeln
Alkyl-Cy-$CH_2CH_2$-Phe-Pyr-Alkyl
Alkyl-Cy-$CH_2O$-Phe-Pyr-Alkyl
worin Cy, Phe und Pyr die in Anspruch 4 gegebene Bedeutung besitzen, und Alkyl jeweils eine geradkettige Alkylgruppe mit 2 bis 10 C-Atomen bedeutet.

9. Verbindungen nach Anspruch 4 entsprechend der Teilformeln
Alkyl-Cy-$CH_2CH_2$-Pyr-Phe-Alkyl
Alkyl-Cy-$CH_2CH_2$-Pyr-Phe-F
Alkyl-Cy-$CH_2CH_2$-Pyr-Phe-CN
worin Cy, Phe und Pyr die in Anspruch 4 gegebene Bedeutung besitzen, und Alkyl jeweils eine geradkettige Alkylgruppe mit 2 bis 10 C-Atomen bedeutet.

10. Verfahren zur Herstellung von Verbindungen der Formel I nach Anspruch 1, dadurch gekennzeichnet, daß man eine Verbindung, die sonst der Formel I entspricht, aber an Stelle von H-Atomen eine oder mehrere reduzierbare Gruppen und/oder C—C-Bindungen enthält, mit einem reduzierenden Mittel behandelt.
oder daß man zur Herstellung von Estern der Formel I (worin $R^1$ und/oder $R^2$ eine Alkylgruppe bedeuten, worin eine oder zwei $CH_2$-Gruppen durch —O—CO-Gruppen und/oder —CO—O-Gruppen ersetzt sind) eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von 1,3-Dioxanderivaten bzw. 1,3-Dithianderivaten der Formel I (worin $A^1$ 1,3-Dioxan-2,5-diyl bzw. 1,3-Dithian-2,5-diyl bedeutet) einen entsprechenden Aldehyd mit einem entsprechenden Diol bzw. Dithiol umsetzt,
oder daß man zur Herstellung von Ethern der Formel I (worin $R^1$ und/oder $R^2$ eine Alkylgruppe bedeuten, worin eine oder zwei $CH_2$-Gruppen durch O-Atome ersetzt sind und/oder Z ein —$OCH_2$— oder —$CH_2$—O-Gruppe ist) eine entsprechenede Hydroxyverbindung verethert,

11. Verwendung der Verbindungen der Formel I nach Anspruch 1 als Komponenten flüssigkristalliner Phasen.

12. Flüssigkristalline Phase mit mindestens zwei flüssigkristallinen Komponenten, dadurch gekennzeichnet, daß mindestens eine Komponente eine Verbindung der Formel I nach Anspruch 1 ist.

13. Flüssigkristallanzeigeelement, dadurch gekennzeichnet, daß es eine Phase nach Anspruch 12 enthält.

14. Elektrooptisches Anzeigeelement nach Anspruch 13, dadurch gekennzeichnet, daß es als Dielektrikum eine Phase nach Anspruch 12 enthält.

**Revendications pour les Etats contractants: CH DE GB LI**

1. Composés de formule I

$$R^1—A^1—Z^1—A^2—Z^2—A^3—R^2$$

dans laquelle

$R^1$ et $R^2$ représentent chacun, indépendamment l'un de l'autre, un groupe alkyle en C1—C15 dans lequel un ou deux groupes $CH_2$ non voisins peuvent être remplacés par des atomes d'oxygène et/ou des groupes —CO— et/ou des groupes —O—CO— et/ou des groupes —CO—O—, l'un des symboles $R^1$ et $R^2$ pouvant également représenter H, F, Cl, Br ou CN,

$A^1$ représente un groupe 1,4-cyclohexylène dans lequel un ou deux groupes $CH_2$ non voisins peuvent être remplacés par des atomes d'oxygène, un groupe 1,4-bicyclo(2,2,2)octylène ou un groupe 1,3-dithianne-2,5-diyle,

$Z^1$ et $Z^2$ représentent chacun indépendamment l'un de l'autre, —$CH_2CH_2$—, —$OCH_2$—, —$CH_2O$— ou une liaison simple, et

$A^2$ et $A^3$ représentent chacun, indépendamment l'un de l'autre, un groupe 1,4-phénylène, un groupe pyrimidine-2,5-diyle, un groupe 1,4-cyclohexylène dans lequel un ou deux groupes $CH_2$ non voisins peuvent être remplacés par des atomes d'oxygène, un groupe 1,3-dithianne-2,5-diyle ou un groupe 1,4-bicyclo(2,2,2)octylène, avec les réserves suivantes:

(a) au moins un des symboles $A^2$ et $A^3$ représente un groupe pyrimidine-2,5-diyle,

(b) au moins un des symboles $Z^1$ et $Z^2$ ne peut représenter une liaison simple lorsque $A^2$ représente un groupe pyrimidine-2,5-diyle et $R^2$ un groupe alkyle ou CN,

(c) sont exclues:

la 2-(4-cyanophényl)-5-[2-(trans-4-éthylcyclohexyl)-éthyl]-pyrimidine,
la 2-(4-cyanophényl)-5-[2-(trans-4-propylcyclohexyl)-éthyl]-pyrimidine,
la 2-(4-cyanophényl)-5-[2-(trans-4-butylcyclohexyl)-éthyl]-pyrimidine,
la 2-(4-cyanophényl)-5-[2-(trans-4-pentylcyclohexyl)-éthyl]-pyrimidine,
la 2-(4-cyanophényl)-5-[2-(trans-4-heptylcyclohexyl)-éthyl]-pyrimidine, et

(d) sont exclues les 5-alkyl-2-[4'-(trans-4''-alkylcyclohexyl)-phényl]-pyrimidines.

2. Composés selon la revendication 1, dans lesquels $R^1$ et $R^2$ représentent chacun, indépendamment l'un de l'autre, un groupe alkyle, alcoxy ou oxa-alkyle à chaîne droite.

3. Composés selon la revendication 1 ou 2, dans lesquels $A^1$ représente un groupe trans-1,4-cyclohexylène.

4. Composés selon l'une des revendications 1 à 3, répondant aux formules partielles Ica, Ifi, Ifk, Ifj et Ifl.

| | |
|---|---|
| $R^1$-Cy-Pyr-Phe-$R^2$ | Ica |
| $R^1$-Cy-$Z^1$-Pyr-Phe-$R^2$ | Ifi |
| $R^1$-Cy-$Z^1$-Phe-Pyr-$R^2$ | Ifk |
| $R^1$-Cy-Pyr-$Z^2$-Phe-$R^2$ | Iij |
| $R^1$-Cy-Phe-$Z^2$-Pyr-$R^2$ | Iil, |

dans lesquelles $R^1$ et $R^2$ ont les significations indiquées ci-dessus, et

Cy représente un groupe 1,4-cyclohexylène,
Pyr représente un groupe pyrimidine-2,5-diyle,
Phe représente un groupe 1,4-phénylène, et
$Z^1$ et $Z^2$ représentent —$CH_2O$—, —$OCH_2$—, ou —$CH_2CH_2$—.

5. Composés selon la revendication 4, répondant aux formules partielles Ica, Ifi, Ifk, Iij et Iil dans lesquelles $R^1$, $Z^1$, $Z^2$, Cy, Pyr et Phe ont les significations indiquées ci-dessus, et

$R^2$ représente F.

6. Composés selon la revendication 4 ou 5, dans lesquels $Z^1$ et/ou $Z^2$ représentent —$CH_2CH_2$— ou —$CH_2O$—.

7. Composé selon la revendication 3, répondant aux formules partielles

Alkyl-Cy-Cy-Pyr-Alkyl
Alkyl-Cy-Cy-Pyr-Alkoxy

dans lesquelles Cy et Pyr ont les significations indiquées dans la revendication 2 et "Alkyl" représente un group alkyle à chaîne droite en C2—C10 et "Alkoxy" un groupe alcoxy à chaîne droit en C2—C12.

8. Composés selon la revendication 4, répondant aux formules partielles

Alkyl-Cy-$CH_2CH_2$-Phe-Pyr-Alkyl
Alkyl-Cy-$CH_2O$-Phe-Pyr-Alkyl

dans lesquelles Cy, Phe et Pyr ont les significations indiquées dans la revendication 4 et chacun des symboles "Alkyl" représente un groupe alkyle à chaîne droite en C2—C10.

9. Composés selon la revendication 4, répondant aux formules partielles

Alkyl-Cy-$CH_2CH_2$-Pyr-Phe-Alkyl
Alkyl-Cy-$CH_2CH_2$-Pyr-Phe-F
Alkyl-Cy-$CH_2CH_2$-Pyr-Phe-CN

dans lesquelles Cy, Phe et Pyr ont les significations indiquées dans la revendication 4, et chacun des symboles "Alkyl" représente un groupe alkyle à chaîne droite en C2—C10.

10. Procédé de préparation des composés de formule I de la revendication 1, caractérisé en ce que l'on traite par un agent réducteur un composé répondant par ailleurs à la formule I mais contenant, à la place d'atomes d'hydrogène, un ou plusieurs groupes réductibles et/ou une ou plusieurs liaisons C—C,

ou bien en ce que, pour préparer les esters de formule I (dans laquelle $R^1$ et/ou $R^2$ représentent des groupes alkyle dans lesquels un ou deux groupes $CH_2$ sont remplacés par des groupes —O—CO— et/ou —CO—O—), on fait réagir un acide carboxylique correspondant ou l'un de ses dérivés réactifs avec un alcool correspondant ou l'un de ses dérivés réactifs,

ou bien en ce que, pour préparer les dérivés du 1,3-dioxanne ou du 1,3-dithianne de formule I (dans laquelle $A^1$ représente respectivement un groupe 1,3-dioxanne-2,5-diyle ou 1,3-dithianne-2,5-diyle) on fait réagir un aldéhyde correspondant avec un diol ou dithiol correspondant respectivement,

ou bien en ce que, pour préparer les éthers de formule I (dans laquelle $R^1$ et/ou $R^2$ représentent des groupes alkyle dans lesquels un ou deux groupes $CH_2$ sont remplacés par des atomes d'oxygène, et/ou Z représente un groupe $—OCH_2—$ ou $—CH_2O—$), on éthérifie un dérivé hydroxylé correspondant,

11. Utilisation des composés de formule I de la revendication 1 en tant que composants de phases à cristaux liquides.

12. Phase à cristaux liquides contenant au moins deux composants à cristaux liquides, caractérisée en ce que l'un au moins des composants est un composé de formule I de la revendication 1.

13. Phase à cristaux liquides à au moins deux composants à cristaux liquides, caractérisée en ce que l'un au moins des composants est un composé de formule I

$$R^1—A^1—Z^1—A^2—Z^2—A^3—R^2 \qquad I$$

dans laquelle $R^1$, $R^2$, $A^1$, $A^2$, $A^3$, $Z^1$ et $Z^2$ ont les significations indiquées dans la revendication 1, avec les réserves suivantes:

(a) l'un au moins des symboles $A^2$ et $A^3$ représente un groupe pyrimidine-2,5-diyle, et

(b) l'un au moins des symboles $Z^1$ et $Z^2$ ne peut représenter un liaison simple lorsque $A^2$ représente un groupe pyrimidine-2,5-diyle et $R^2$ un groupe alkyle ou CN,

(c) sont exclues:

la 2-(4-cyanophényl)-5-[2-(trans-4-propylcyclohexyl)éthyl]-pyrimidine,
la 2-(4-cyanophényl)-5-[2-(trans-4-propylcyclohexyl)éthyl]-pyrimidine,
la 2-(4-cyanophényl)-5-[2-(trans-4-butylcyclohexyl)éthyl]-pyrimidine,
la 2-(4-cyanophényl)-5-[2-(trans-4-pentylcyclohexyl)éthyl]-pyrimidine,
la 2-(4-cyanophényl)-5-[2-(trans-4-heptylcyclohexyl)éthyl]-pyrimidine,

et la phase contient au moins un constituant de formula II

$$R'—L—G—E—R'' \qquad II$$

dans laquelle

L et E représentent chacun un système carbocyclique ou hétérocyclique pris dans le groupe formé par les cycles benzène et cyclohexane 1,4-disubstitués, les systèmes biphényle, phénylcyclohexane et cyclohexylcyclohexane 4,4'-disubstitués, les cycles pyrimidine et 1,3-dioxanne 2,5-disubstitués, le naphtalène 2,6-disubstitué, le diet le tétrahydronaphtalène, la quinazoline et la tétrahydroquinazoline,

| G représente | | |
|---|---|---|
| | —CH=CH— | —N(O)=N— |
| | —CH=CY— | —CH=N(O)— |
| | —CH≡C— | $—CH_2—CH_2—$ |
| | —CO—O— | $—CH_2—O—$ |
| | —CO—S— | $—CH_2—S—$ |
| | —CH=N— | —COO—Ph—COO— |

Y représente un halogène ou un groupe —CN, et

R' et R'' représentent des groupes alcoxy, alcanoyloxy ou alcoxycarbonyloxy contenant jusqu'à 8 atomes de carbone, l'un des ces symboles pouvant également représenter CN, NC, $NO_2$, $CF_3$, F, Cl ou Br, ou bien elle contient au moins un autre constituant choisi dans les classes des benzylidèneanilines, des terphényles, des cyclohexylbiphényles, des cyclohexylcyclohexanes, des cyclohexylnaphtalènes, des 1,4-bis-cyclohexylbenzènes, des 4,4'-bis-cyclohexylbiphényles, des stilbènes éventuellement halogénés, des éthers benzylphényliques, des tolanes et des acides cinnamiques substitués.

14. Elément d'affichage à cristaux liquides, caractérisé en ce qu'il contient une phase selon au moins une des revendications 12 et 13.

15. Elément d'affichage électro-optique selon la revendication 14, caractérisé en ce qu'il contient en tant que diélectrique une phase selon au moins une des revendications 12 et 13.

**Revendications pour l'Etat contractant: FR**

1. Composé de formule I

$$R^1—A^1—Z^1—A^2—Z^2—A^3—R^2$$

dans laquelle

$R^1$ et $R^2$ représentent chacun, indépendamment l'un de l'autre, un groupe alkyle en C1—C15 dans lequel un ou deux groupes $CH_2$ non voisins peuvent être remplacés par des atomes d'oxygène et/ou des groupes —CO— et/ou des groupes —O—CO— et/ou des groupes —CO—O—, l'un des symboles $R^1$ et $R^2$ pouvant également représenter H, F, Cl ou Br,

$A^1$ représente un groupe 1,4-cyclohexylène dans lequel un ou deux groupes $CH_2$ non voisins peuvent être remplacés par des atomes d'oxygène, un groupe 1,4-bicyclo(2,2,2)octylène ou un groupe 1,3-dithianne-2,5-diyle,

$Z^1$ et $Z^2$ représentent chacun indépendamment l'un de l'autre, un groupe $—CH_2CH_2—$, $—OCH_2—$, $—CH_2O—$ ou une liaison simple, et

$A^2$ et $A^3$ représentent chacun, indépendamment l'un de l'autre, un groupe 1,4-phénylène, un groupe pyrimidine-2,5-diyle, un groupe 1,4-cyclohexylène dans lequel un ou deux groupes $CH_2$ non voisins peuvent être remplacés par des atomes d'oxygène, un groupe 1,3-dithianne-2,5-diyle ou un groupe 1,4-bicyclo(2,2,2)octylène, avec les réserves suivantes:

(a) au moins un des symboles $A^2$ et $A^3$ représente un groupe pyrimidine-2,5-diyle,

(b) au moins un des symboles $Z^1$ et $Z^2$ ne peut représenter une liaison simple lorsque $A^2$ représente un groupe pyrimidine-2,5-diyle et $R^2$ un groupe alkyle ou CN,

(c) sont exclues:

la 2-(4-cyanophényl)-5-[2-(trans-4-éthylcyclohexyl)-éthyl]-pyrimidine,
la 2-(4-cyanophényl)-5-[2-(trans-4-propylcyclohexyl)-éthyl]-pyrimidine,
la 2-(4-cyanophényl)-5-[2-(trans-4-butylcyclohexyl)-éthyl]-pyrimidine,
la 2-(4-cyanophényl)-5-[2-(trans-4-pentylcyclohexyl)-éthyl]-pyrimidine,
la 2-(4-cyanophényl)-5-[2-(trans-4-heptylcyclohexyl)-éthyl]-pyrimidine.

2. Composés selon la revendication 1, dans lesquels $R^1$ et $R^2$ représentent chacun, indépendamment l'un de l'autre, un groupe alkyle, alcoxy ou oxa-alkyle à chaîne droite.

3. Composés selon la revendication 1 ou 2, dans lesquels $A^1$ représente un group trans-1,4-cyclohexylène.

4. Composés selon l'une des revendications 1 à 3, répondant aux formules partielles Ica, Ifi, Ifk, Ifj et Ifl.

| | |
|---|---|
| $R^1$-Cy-Pyr-Phe-$R^2$ | Ica |
| $R^1$-Cy-$Z^1$-Pyr-Phe-$R^2$ | Ifi |
| $R^1$-Cy-$Z^1$-Phe-Pyr-$R^2$ | Ifk |
| $R^1$-Cy-Pyr-$Z^2$-Phe-$R^2$ | Iij |
| $R^1$-Cy-Phe-$Z^2$-Pyr-$R^2$ | Iil, |

dans lesquelles $R^1$ et $R^2$ ont les significations indiquées ci-dessus, et

Cy représente un groupe 1,4-cyclohexylène,
Pyr représente un groupe pyrimidine-2,5-diyle,
Phe représente un groupe 1,4-phénylène, et
$Z^1$ et $Z^2$ représentent $—CH_2O—$, $—OCH_2—$, ou $—CH_2CH_2—$.

5. Composés selon la revendication 4, répondant aux formules partielles Ica, Ifi, Ifk, Iij et Iil dans lesquelles $R^1$, $Z^1$, $Z^2$, Cy, Pyr et Phe ont les significations indiquées ci-dessus, et

$R^2$ représente F.

6. Composés selon la revendication 4 ou 5, dans lesquels $Z^1$ et/ou $Z^2$ représentent $—CH_2CH_2—$ ou $—CH_2O—$.

7. Composé selon la revendication 3, répondant aux formules partielles

Alkyl-Cy-Cy-Pyr-Alkyl
Alkyl-Cy-Cy-Pyr-Alkoxy

dans lesquelles Cy et Pyr ont les significations indiquées dans la revendication 2 et "Alkyl" représente un group alkyle à chaîne droite en C2—C10 et "Alkoxy" un groupe alcoxy à chaîne droite en C2—C12.

8. Composés selon la revendication 4, répondant aux formules partielles

Alkyl-Cy-$CH_2CH_2$-Phe-Pyr-Alkyl
Alkyl-Cy-$CH_2O$-Phe-Pyr-Alkyl

dans lesquelles Cy, Phe et Pyr ont les significations indiquées dans la revendication 4 et chacun des symboles "Alkyl" représente un groupe alkyle à chaîne droite en C2—C10.

9. Composés selon la revendication 4, répondant aux formules partielles

Alkyl-Cy-$CH_2CH_2$-Pyr-Phe-Alkyl
Alkyl-Cy-$CH_2CH_2$-Pyr-Phe-F
Alkyl-Cy-$CH_2CH_2$-Pyr-Phe-CN

dans lesquelles Cy, Phe et Pyr ont les significations indiquées dans la revendication 4, et chacun des symboles "Alkyl" représente un groupe alkyle à chaîne droite en C2—C10.

10. Procédé de préparation des composés de formule I de la revendication 1, caractérisé en ce que l'on traite par un agent réducteur un composé répondant par ailleurs à la formule I mais contenant, à la place d'atomes d'hydrogène, un ou plusieurs groupes réductibles et/ou une ou plusieurs liaisons C—C,

ou bien en ce que, pour préparer les esters de formule I (dans laquelle $R^1$ et/ou $R^2$ représent des groupes alkyle dans lesquels un ou deux groupes $CH_2$ sont remplacés par des groupes —O—CO— et/ou —CO—O—), on fait réagir un acide carboxylique correspondant ou l'un de ses dérivés réactifs avec un alcool correspondant ou l'un de ses dérivés réactifs,

ou bien en ce que, pour préparer les dérivcés du 1,3-dioxanne ou du 1,3-dithianne de formule I (dans laquelle $A^1$ représente respectivement un groupe 1,3-dioxanne-2,5-diyle ou 1,3-dithianne-2,5-diyle) on fait réagir un aldéhyde correspondant avec un diol ou dithiol correspondant respectivement,

ou bien en ce que, pour préparer les éthers de formule I (dans laquelle $R^1$ et/ou $R^2$ représentent des groupes alkyle dans lesquels un ou deux groupes $CH_2$ sont remplacés par des atomes d'oxygène, et/ou Z représente un groupe —$OCH_2$— ou —$CH_2O$—), on éthérifie un composé hydroxylé correspondant,

11. Utilisation des composés de formule I de la revendication 1 en tant que composants de phases à cristaux liquides.

12. Phase à cristaux liquides contenant au moins deux composants à cristaux liquides, caractérisée en ce que l'un au moins des composants es un composé de formule I de la revendication 1.

13. Elément d'affichage à cristaux liquides, caractérisé en ce qu'il conteint une phase selon la revendication 12.

14. Elément d'affichage électro-optique selon la revendication 13, caractérisé en ce qu'il contient en tant que diélectrique une phase selon la revendication 12.

**Claims for the Contracting States: CH DE GB LI**

1. Compounds of the formula I

$$R^1—A^1—Z^1—A^2—Z^2—A^3—R^2$$

wherein

$R^1$ and $R^2$ in each case independently of one another are an alkyl group having 1—15 C atoms, it also being possible for one or two non-adjacent $CH_2$ groups to be replaced by O atoms and/or —CO— groups and/or —O—CO— groups and/or —CO—O— groups, and one of the radicals $R^1$ and $R^2$ is also H, F, Cl, Br or CN,

$A^1$ is a 1,4-cyclohexylene group, it also being possible for one or two non-adjacent $CH_2$ groups to be replaced by O atoms, a 1,4-bicyclo-(2,2,2)-octylene group or a 1,3-dithiane-2,5-diyl group,

$Z^1$ and $Z^2$ in each case independently of one another are —$CH_2CH_2$—, —$OCH_2$—, —$CH_2O$— or a single bond, and

$A^2$ and $A^3$ in each case independently of one another are a 1,4-phenylene group, pyrimidine-2,5-diyl group, 1,4-cyclohexylene group, it also being possible for one or two non-adjacent $CH_2$ groups to be replaced by O atoms, 1,3-dithiane-2,5-diyl group or a 1,4-bicyclo(2,2,2)-octylene group,

with the provisos that

(a) at least one of the groups $A^2$ and $A^3$ is a pyrimidine-2,5-diyl group,

(b) at least one of the groups $Z^1$ and $Z^2$ is not a single bond if $A^2$ is pyrimidine-2,5-diyl and $R^2$ is alkyl or CN,

(c) 2-(4-cyanophenyl)-5-[2-(trans-4-ethylcyclohexyl)-ethyl]-pyrimidine,
2-(4-cyanophenyl)-5-[2-(trans-4-propylcyclohexyl)-ethyl]-pyrimidine,
2-(4-cyanophenyl)-5-[2-(trans-4-butylcyclohexyl)-ethyl]-pyrimidine,
2-(4-cyanophenyl)-5-[2-(trans-4-pentylcyclohexyl)-ethyl]-pyrimidine, and
2-(4-cyanophenyl)-5-[2-(trans-4-heptylcyclohexyl)-ethyl]-pyrimidine,

is (sic) excluded, and

(d) 5-alkyl-2-(4'-(trans-4''-alkylcyclohexyl)phenyl)pyrimidines are excluded.

2. Compounds according to Claim 1, wherein $R^1$ and $R^2$ in each case independently of one another are a straight-chain alkyl, alkoxy or oxaalkyl group.

3. Compounds according to Claim 1 or 2, wherein

$A^1$ is a trans-1,4-cyclohexylene group.

4. Compounds according to one of Claims 1 to 3 corresponding to the part formulae Ica, Ifi, Ifk, Ifj and Ifl

| | |
|---|---|
| $R^1$-Cy-Pyr-Phe-$R^2$ | Ica |
| $R^1$-Cy-$Z^1$-Pyr-Phe-$R^2$ | Ifi |
| $R^1$-Cy-$Z^1$-Phe-Pyr-$R^2$ | Ifk |

$R^1$-Cy-Pyr-$Z^2$-Phe-$R^2$ Iij
$R^1$-Cy-Phe-$Z^2$-Pyr-$R^2$ Iil,

wherein $R^1$ and $R^2$ have the meaning given and
Cy is a 1,4-cyclohexylene group,
Pyr is a pyrimidine-2,5-diyl group,
Phe is a 1,4-phenylene group and
$Z^1$ and $Z^2$ are $—CH_2O—$, $—OCH_2—$ or $—CH_2CH_2—$.

5. Compounds according to Claim 4 corresponding to the part formulae Ica, Ifi, Ifk, Iij and Iil, wherein $R^1$, $Z^1$, $Z^2$, Cy, Pyr and Phe have the meaning given, and
$R^2$ is F.

6. Compounds according to Claim 4 or 5, wherein $Z^1$ or $Z^2$ is $—CH_2CH_2—$ or $—CH_2O—$.

7. Compounds according to Claim 3 corresponding to the part formulae
Alkyl-Cy-Cy-Pyr-Alkyl
Alkyl-Cy-Cy-Pyr-Alkoxy
wherein Cy and Pyr have the meaning given in Claim 2 and alkyl is a straight-chain alkyl group having 2 to 10 C atoms and alkoxy is a straight-chain alkoxy group having 2 to 12 C atoms.

8. Compounds according to Claim 4 corresponding to the part formulae
Alkyl-Cy-$CH_2CH_2$-Phe-Pyr-Alkyl
Alkyl-Cy-$CH_2O$-Phe-Pyr-Alkyl
wherein Cy, Phe and Pyr have the meaning given in Claim 4 and alkyl in each case is a straight-chain alkyl group having 2 to 10 C atoms.

9. Compounds according to Claim 4 corresponding to the part formulae
Alkyl-Cy-$CH_2CH_2$-Pyr-Phe-Alkyl
Alkyl-Cy-$CH_2CH_2$-Pyr-Phe-F
Alkyl-Cy-$CH_2CH_2$-Pyr-Phe-CN
wherein Cy, Phe and Pyr have the meaning given in Claim 4 and alkyl in each case is a straight-chain alkyl group having 2 to 10 C atoms.

10. Process for the preparation of compounds of the formula I according to Claim 1, characterized in that a compound which otherwise corresponds to the formula I but contains one or more reducible groups and/or C—C bonds instead of H atoms is treated with a reducing agent, or in that, to prepare esters of the formula I (wherein $R^1$ and/or $R^2$ are an alkyl group, in which one ore two $CH_2$ groups are replaced by —O—CO— groups and/or —CO—O— groups), a corresponding carboxylic acid or one of its reactive derivatives is reacted with a corresponding alcohol or one of its reactive derivatives, or in that, to prepare 1,3-dioxane derivatives or 1,3-dithiane derivatives of the formula I (wherein $A^1$ is 1,3-dioxane-2,5-diyl or 1,3-dithiane-2,5-diyl), a corresponding aldehyde is reacted with a corresponding diol or dithiol, or in that, to prepare ethers of the formula I (wherein $R^1$ and/or $R^2$ are an alkyl group, in which one or two $CH_2$ groups are replaced by O atoms, and/or Z is an $—OCH_2—$ or $—CH_2O—$ group), a corresponding hydroxy compound is etherified.

11. Use of the compounds of the formula I according to Claim 1 as components of liquid crystal phases.

12. Liquid crystal phase containing at least two liquid crystal components characterized in that at least one component is a compound of the formula I according to Claim 1.

13. Liquid crystal phase containing at least two liquid crystal components, characterized in that at least one component is a compound of the formula I

$$R^1—A^1—Z—A^2—Z^2—A^3—R^2 \qquad I$$

wherein $R^1$, $R^2$, $A^2$, $A^2$, $A^3$, $Z^1$ and $Z^2$ have the meaning given in Claim 1, with the provisos that
(a) at least one of the groups $A^2$ and $A^3$ is a pyrimidine-2,5-diyl group, and
(b) at least one of the groups $Z^1$ and $Z^2$ is not a single bond if $A^2$ is pyrimidine-2,5-diyl and $R^2$ is alkyl or CN, and
(c) 2-(4-cyanophenyl)-5-[2-(trans-4-ethylcyclohexyl)-ethyl]pyrimidine,
2-(4-cyanophenyl)-5-[2-(trans-4-propylcyclohexyl)-ethyl]-pyrimidine,
2-(4-cyanophenyl)-5-[2-(trans-4-butylcyclohexyl)-ethyl]-pyrimidine,
2-(4-cyanophenyl)-5-[2-(trans-4-pentylcyclohexyl)-ethyl]-pyrimidine and
2-(4-cyanophenyl)-5-[2-(trans-4-heptylcyclohexyl)-ethyl]-pyrimidine, is (sic) excluded, and it contains (sic) at least one constituent of the formula II

$$R'—L—G—E—R'' \qquad II$$

wherein
L and E are each a carbo- or heterocyclic ring system from the group formed by 1,4-disubstituted benzene and cyclohexane rings, 4,4'-disubstituted biphenyl, phenylcyclohexane and cyclohexylcyclohexane systems, 2,5-disubstituted pyrimidine and 1,3-dioxane rings, 2,6-disubstituted naphthalene, di- and tetrahydronaphthalene, quinazoline and tetrahydroquinazoline,

G is the formulae

| | |
|---|---|
| —CH=CH— | —N(O)=N— |
| —CH=CY— | —CH=N(O)— |
| —CH≡C— (sic) | $—CH_2CH_2—$ |
| —CO—O— | $—CH_2—O—$ |
| —CO—S— | $—CH_2—S—$ |
| —CH=N— | —COO—Ph—COO— |

Y is halogen or —CN and

R′ and R″ are alkoxy, alkanoyloxy or alkoxycarbonyloxy having up to 8 carbon atoms, or one of these radicals is also CN, NC, $NO_2$, $CF_3$, F, Cl or Br, or it contains (sic) at least one other constituent selected from the classes of benzylideneanilines, terphenyls, cyclohexylbiphenyls, cyclohexylcyclohexanes, cyclohexylnaphthalenes, 1,4-bis-cyclohexylbenzenes, 4,4′-bis-cyclohexylbiphenyls, optionally halogenated stilbenes, benzyl phenyl ethers, tolanes and substituted cinnamic acids.

14. Liquid crystal display element, characterized in that it contains a phase according to at least one of Claims 12 and 13.

15. Electro-optical display element according to Claim 14, characterized in that it contains a phase according to at least one of claims 12 and 13 as the dielectric.

**Claims for the Contracting State: FR**

1. Compounds of the formula I

$$R^1—A^1—Z—A^2—Z^2—A^3—R^2$$

wherein

$R^1$ and $R^2$ in each case independently of one another are an alkyl group having 1—15 C atoms; it also being possible for one or two non-adjacent $CH_2$ groups to be replaced by O atoms and/or —CO— groups and/or —O—CO— grops and/or —CO—O— groups, and one of the radicals $R^1$ and $R^2$ is also H, F, Cl, or Br,

$A^1$ is a 1,4-cyclohexylene group, it also being possible for one or two non-adjacent $CH_2$ groups to be replaced by O atoms, a 1,4-bicyclo-(2,2,2)-octylene group or a 1,3-dithiane-2,5-diyl group,

$Z^1$ and $Z^2$ in each case independently of one another are $—CH_2CH_2—$, $—OCH_2—$, $—CH_2O—$ or a single bond, and

$A^2$ and $A^3$ in each case independently of one another are a 1,4-phenylene group, pyrimidine-2,5-diyl group, 1,4-cyclohexylene group, it also being possible for one or two non-adjacent $CH_2$ groups to be replaced by O atoms, 1,3-dithiane-2,5-diyl group or a 1,4-bicyclo(2,2,2)-octylene group,

with the provisos that

(a) at least one of the groups $A^2$ and $A^3$ is a pyrimidine-2,5-diyl group,

(b) at least one of the groups $Z^1$ and $Z^2$ is not a single bond if $A^2$ is pyrimidine-2,5-diyl and $R^2$ is alkyl or CN,

(c) 2-(4-cyanophenyl)-5-[2-(trans-4-ethylcyclohexyl)-ethyl]-pyrimidine,
2-(4-cyanophenyl)-5-[2-(trans-4-propylcyclohexyl)-ethyl]-pyrimidine,
2-(4-cyanophenyl)-5-[2-(trans-4-butylcyclohexyl)-ethyl]-pyrimidine,
2-(4-cyanophenyl)-5-[2-(trans-4-pentylcyclohexyl)-ethyl]-pyrimidine, and
2-(4-cyanophenyl)-5-[2-(trans-4-heptylcyclohexyl)-ethyl]-pyrimidine,

is (sic) excluded.

2. Compounds according to Claim 1, wherein $R^1$ and $R^2$ in each case independently of one another are a straight-chain alkyl, alkoxy or oxaalkyl group.

3. Compounds according to Claim 1 or 2, wherein

$A^1$ is a trans-1,4-cyclohexylene group.

4. Compounds according to one of Claims 1 to 3 corresponding to the part formulae Ica, Ifi, Ifk, Ifj and Ifl

| | |
|---|---|
| $R^1$-Cy-Pyr-Phe-$R^2$ | Ica |
| $R^1$-Cy-$Z^1$-Pyr-Phe-$R^2$ | Ifi |
| $R^1$-Cy-$Z^1$-Phe-Pyr-$R^2$ | Ifk |
| $R^1$-Cy-Pyr-$Z^2$-Phe-$R^2$ | Iij |
| $R^1$-Cy-Phe-$Z^2$-Pyr-$R^2$ | Iil, |

wherein $R^1$ and $R^2$ have the meaning given and

Cy is a 1,4-cyclohexylene group,

Pyr is a pyrimidine-2,5-diyl group,

Phe is a 1,4-phenylene group and

$Z^1$ and $Z^2$ are $—CH_2O—$, $—OCH_2—$ or $—CH_2CH_2—$.

5. Compounds according to Claim 4 corresponding to the part formulae Ica, Ifi, Ifk, Iij and Iil, wherein $R^1$, $Z^1$, $Z^2$, Cy, Pyr and Phe have the meaning given, and

$R^2$ is F.

6. Compounds according to Claim 4 or 5, wherein $Z^1$ or $Z^2$ is $—CH_2CH_2—$ or $—CH_2O—$.

7. Compounds according to Claim 3 corresponding to the part formulae

Alkyl-Cy-Cy-Pyr-Alkyl

Alkyl-Cy-Cy-Pyr-Alkoxy

wherein Cy and Pyr have the meaning given in Claim 2 and alkyl is a straight-chain alkyl group having 2 to 10 C atoms and alkoxy is a straight-chain alkoxy group having 2 to 12 C atoms.

8. Compounds according to Claim 4 corresponding to the part formulae

Alkyl-Cy-$CH_2CH_2$-Phe-Pyr-Alkyl

Alkyl-Cy-$CH_2O$-Phe-Pyr-Alkyl

wherein Cy, Phe and Pyr have the meaning given in Claim 4 and alkyl in each case is a straight-chain alkyl group having 2 to 10 C atoms.

9. Compound according to Claim 4 corresponding to the part formulae

Alkyl-Cy-$CH_2CH_2$-Pyr-Phe-Alkyl

Alkyl-Cy-$CH_2CH_2$-Pyr-Phe-F

Alkyl-Cy-$CH_2CH_2$-Pyr-Phe-CN

wherein Cy, Phe and Pyr have the meaning given in Claim 4 and alkyl in each case is a straight-chain alkyl group having 2 to 10 C atoms.

10. Process for the preparation of compounds of the formula I according to Claim 1, characterized in that a compound which otherwise corresponds to the formula I but contains one or more reducible groups and/or C—C bonds instead of H atoms is treated with a reducing agent, or in that, to prepare esters of the formula I (wherein $R^1$ and/or $R^2$ are an alkyl group, in which one or two $CH_2$ groups are replaced by —O—CO— groups and/or —CO—O— groups), a corresponding carboxylic acid or one of its reactive derivatives is reacted with a corresponding alcohol or one of its reactive derivatives, or in that, to prepare 1,3-dioxane derivatives or 1,3-dithiane derivatives of the formula I (wherein $A^1$ is 1,3-dioxane-2,5-diyl or 1,3-dithiane-2,5-diyl), a corresponding aldehyde is reacted with a corresponding diol or dithiol, or in that, to prepare ethers of the formula I (wherein $R^1$ and/or $R^2$ are an alkyl group, in which one or two $CH_2$ groups are replaced by O atoms, and/or Z is an $—OCH_2—$ or $—CH_2O—$ group), a corresponding hydroxy compound is etherified.

11. Use of the compounds of the formula I according to Claim 1 as components of liquid crystal phases.

12. Liquid crystal phase containing at least two liquid crystal components, characterized in that at least one component is a compound of the formula I according to Claim 1.

13. Liquid crystal display element, characterized in that it contains a phase according to Claim 12.

14. Electrooptical display element according to Claim 13, characterized in that it contains a phase according to Claim 12 as the dielectric.